(19) Europäisches Patentamt
European Patent Office
Office européen des brevets

(11) **EP 2 904 957 A1**

(12) **EUROPEAN PATENT APPLICATION**
published in accordance with Art. 153(4) EPC

(43) Date of publication:
**12.08.2015 Bulletin 2015/33**

(21) Application number: **14760032.4**

(22) Date of filing: **19.02.2014**

(51) Int Cl.:
***A61B 1/00*** *(2006.01)* ***A61B 1/267*** *(2006.01)*
***A61B 1/273*** *(2006.01)*

(86) International application number:
**PCT/JP2014/053875**

(87) International publication number:
**WO 2014/136576 (12.09.2014 Gazette 2014/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.03.2013 JP 2013044601**

(71) Applicant: **OLYMPUS CORPORATION**
**Shibuya-ku**
**Tokyo 151-0072 (JP)**

(72) Inventors:
- **ITO Mitsuhiro**
  **Tokyo 151-0072 (JP)**
- **AKIMOTO Syunya**
  **Tokyo 151-0072 (JP)**
- **ONISHI Junichi**
  **Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

# (54) ENDOSCOPE SYSTEM

(57) An endoscope system includes: an image recording section that records three dimensional image information of a subject; a luminal organ extracting section that extracts a predetermined luminal organ from the three dimensional image information; a virtual endoscopic image generating section that generates a virtual endoscopic image which is endoscopically drawn from a predetermined viewpoint position with respect to information of the predetermined luminal organ; an image pickup section that picks up an image of an inside of the predetermined luminal organ; a position information obtaining section that obtains position information of a distal end of an insertion portion; a distance comparing section that compares a distance from a feature region in the extracted predetermined luminal organ to the position of the distal end of the insertion portion with a set distance; a variation amount detecting section that detects a variation amount of a feature part of a predetermined luminal organ in the picked-up endoscopic image; and an information recording section that records information including the position of the distal end of the insertion portion and a virtual endoscopic image corresponding to the position based on a result of the comparison by the distance comparing section and a result of the detection by the variation amount detecting section.

**FIG. 2A**

11
Pj
2
da
Bi
2a
35
dth
Bi+1
Pk
K
Bi+2
36

EP 2 904 957 A1

## Description

Technical Field

The present invention relates to an endoscope system that picks up an image of an inside of a subject via image pickup means.

Background Art

In recent years, endoscopes including an insertion portion that can be inserted into, e.g., a body cavity have widely been used in, e.g., medical fields.

On the other hand, when the insertion portion is inserted into a luminal organ that intricately diverges like a bronchus in a body cavity to examine (a diseased tissue of) a target site on the peripheral side of the luminal organ or perform biopsy or treatment of the target site using a treatment instrument, it is sometimes difficult to introduce a distal end of the insertion portion to the vicinity of the target site only by referring to an endoscopic image obtained as a result of the insertion of the insertion portion.

Therefore, systems or apparatuses for supporting an operation to introduce a distal end of an insertion portion of an endoscope to the vicinity of a target site have been proposed.

For example, as a first conventional example, International Publication No. 2007-129493 discloses a configuration of a medical image observation support apparatus including a CT-image-data retrieving section, a CT-image-data storing section, an information extracting section, an anatomical information database, a point of view/line of view direction setting section, a luminal organ image generating section, an anatomical nomenclature information generating section, a branch specifying section, an image synthesizing and displaying section and a user I/F control section. The point of view/line of view setting section locks a point of view on to a substantial center axis of a luminal organ based on structure information of the luminal organ extracted by the information extracting section, to set a point of view and a line of view for observing an appearance of the luminal organ.

Also, as a second conventional example, Japanese Patent Application Laid-Open Publication No. 2011-212244 discloses an endoscope system in which a virtual field of view determining section determines a virtual field of view of a virtual endoscope disposed at a position in a three-dimensional medical image corresponding to a position of an endoscope detected by an endoscope position and posture detecting section based on a position of a structure of interest identified by a position of interest identifying section, a corresponding position and posture of the endoscope and an angle of view of the endoscope obtained by an endoscope angle of view obtaining section so that the position of the structure of interest is included in the virtual field of view and is continuous with a field of view of the endoscope, a virtual endoscopic image generating section receives an input of a three-dimensional medical image formed by a three-dimensional medical image forming section and generates a virtual endoscopic image with the corresponding position of the endoscope set as a point of view, the virtual endoscopic image having the determined virtual field of view, and a display control section makes the generated virtual endoscopic image be displayed on a WS display.

When a position of a distal end of an insertion portion of an endoscope is estimated, the estimation is conducted by comparison between an endoscopic image (actual image) picked up by image pickup means of the endoscope and a virtual endoscopic image (virtual image) generated based on three-dimensional data of a luminal organ provided by CT. Thus, firstly, alignment is performed by comparison between both images.

Then, if the accuracy of estimation of the position is lowered, realignment is required for ensuring a predetermined accuracy. However, the above-mentioned conventional example has a demerit that it takes time to perform realignment.

To explain more specifically, the first conventional example, for example, discloses a perspective to subject the position of the distal end of the endoscope to coordinate transformation on a three-dimensional image and compare the distance from the resultant position to the center line on an actual image and the distance on a virtual image, and a perspective to compare an actual image and a virtual image of a diverging part, but does not expressly disclose recording an amount of information suitable for performing realignment.

The second conventional example also does not disclose recording an amount of information suitable for performing realignment.

As such, it is often the case in conventional examples that there are too many virtual images as comparison candidates, which causes the demerit of taking time for realignment. It is thus desired to record an amount of information that is suitable for realignment. In addition, since it is difficult in conventional examples to visually check the condition for recording, it is desired to allow for recording under a condition that is easy to check to improve convenience for use for the users.

The present invention has been made in view of the above-mentioned points, and an objective of the present invention is to provide an endoscope system that records an amount of information that is suitable for performing realignment under a condition which is easy to visually grasp.

Disclosure of Invention

Means for Solving the Problem

An endoscope system according to an aspect of the present invention includes: an image recording section that records three dimensional image information of a subject obtained in advance; a luminal organ extracting section that extracts a predetermined luminal organ from the three dimensional image information; a virtual endoscopic image generating section that generates a virtual endoscopic image, the virtual endoscopic image being endoscopically drawn from a predetermined viewpoint position with respect to information of the predetermined luminal organ extracted by the luminal organ extracting section; an image pickup section provided inside an endoscope, the image pickup section picking up an image of an inside of the predetermined luminal organ; a position information obtaining section that obtains information on a position of a distal end of an insertion portion of the endoscope as position information; a distance comparing section that compares a distance from a feature region in the predetermined luminal organ extracted by the luminal organ extracting section to the position of the distal end of the insertion portion of the endoscope obtained by the position information obtaining section with a set distance; a variation amount detecting section that detects a variation amount of a feature part of the predetermined luminal organ in the endoscopic image picked up by the image pickup section; and an information recording section that records position and image information including the position of the distal end of the insertion portion of the endoscope and the virtual endoscopic image corresponding to the position of the distal end based on a result of the comparison by the distance comparing section and a result of the detection by the variation amount detecting section.

Brief Description of the Drawings

Fig. 1 is a diagram illustrating an overall configuration of an endoscope system according to a first embodiment of the present invention;
Fig. 2A is a diagram illustrating a part of a bronchus and a bronchus shape image;
Fig. 2B is a diagram illustrating inserting an insertion portion into a bronchus and serially calculating a bronchus diameter;
Fig. 2C is a diagram illustrating positions for which the bronchus diameter was calculated and sizes of the calculated bronchus diameters;
Fig. 2D is a diagram illustrating candidate information pieces displayed on a monitor when an instruction to perform realignment is provided;
Fig. 3A is a diagram illustrating a configuration of an endoscope apparatus including a stereo endoscope that performs stereo measurement;
Fig. 3B is an illustration diagram indicating a relationship where an image of a measurement target position subjected to stereo measurement is formed on an image pickup surface of each of left and right image pickup devices;
Fig. 3C is a diagram illustrating an example in which an image of the inside of a bronchus picked up using a stereo endoscope is displayed on a monitor screen;
Fig. 3D is an illustration diagram for calculating a bronchus diameter from the image in Fig. 3C;
Fig. 3E is an illustration diagram for calculating a bronchus diameter from stereo measurement using a single image pickup apparatus;
Fig. 4A is a flowchart illustrating an example of contents of processing in the first embodiment;
Fig. 4B is a flowchart illustrating details of a part of the contents of processing in Fig. 4A;
Fig. 5 includes diagrams illustrating examples of calculation (measurement) of a distance between a position of a distal end of the insertion portion and a diverging point;
Fig. 6 is a diagram illustrating examples of calculating (measuring) a bronchus diameter;
Fig. 7 is a diagram illustrating a state in which the insertion portion is inserted with a set distance set for a distance between a position of the distal end of the insertion portion and a spur;
Fig. 8 is a flowchart illustrating a part of contents of processing in the case of Fig. 7;
Fig. 9A is a diagram illustrating an example in which a distance between a position of the distal end of the insertion portion and a spur is calculated so as to be a shortest distance;
Fig. 9B is a diagram illustrating an example in which a distance between a position on a center line that is a shortest distance from a position of the distal end of the insertion portion and a spur is calculated;
Fig. 9C is a diagram illustrating an example in which a distance is calculated along a coordinate plane in three-dimensional data;
Fig. 10 is an illustration diagram of a case where the insertion portion is inserted while a distance between a position of the distal end of the insertion portion and the center line is monitored;
Fig. 11A is an illustration diagram of a case where, e.g., a distance between a position of the distal end of the insertion portion and the center line is calculated;
Fig. 11B is an illustration diagram of a case where the distance is calculated according to a method that is different from that in Fig. 11A;
Fig. 12 is an illustration diagram of a case where a user sets set regions and inserts the insertion portion;
Fig. 13 is an illustration diagram of operation of monitoring amounts of variation in brightness from, e.g., the area of a dark part in an endoscopic image when the insertion portion is inserted into a bronchus;
Fig. 14 is an illustration diagram of operation of monitoring amounts of variation in divergence shape of a bronchus in an endoscopic image when the insertion portion is inserted into a bronchus;
Fig. 15 is a diagram illustrating operation of monitoring amounts of variation in length of a spur in an endoscopic image when the insertion portion is inserted into a bronchus, and variation of the length of the spur when a position of the distal end of the insertion portion is moved;

Fig. 16 is a diagram illustrating operation of monitoring amounts of variation in angle of a spur in an endoscopic image when the insertion portion is inserted into a bronchus, and variation of the angle of the spur;

Fig. 17 is an illustration diagram of operation of monitoring occurrence of poor visibility in an endoscopic image when the insertion portion is inserted into a bronchus;

Fig. 18 is an illustration diagram of operation of monitoring variation to a shape other than a divergence in an endoscopic image when the insertion portion is inserted into a bronchus; and

Fig. 19 is an illustration diagram of a configuration that calculates an amount of displacement in an endoscopic image.

Best Mode for Carrying Out the Invention

An embodiment of the present invention will be described below with reference to drawings.

(First Embodiment)

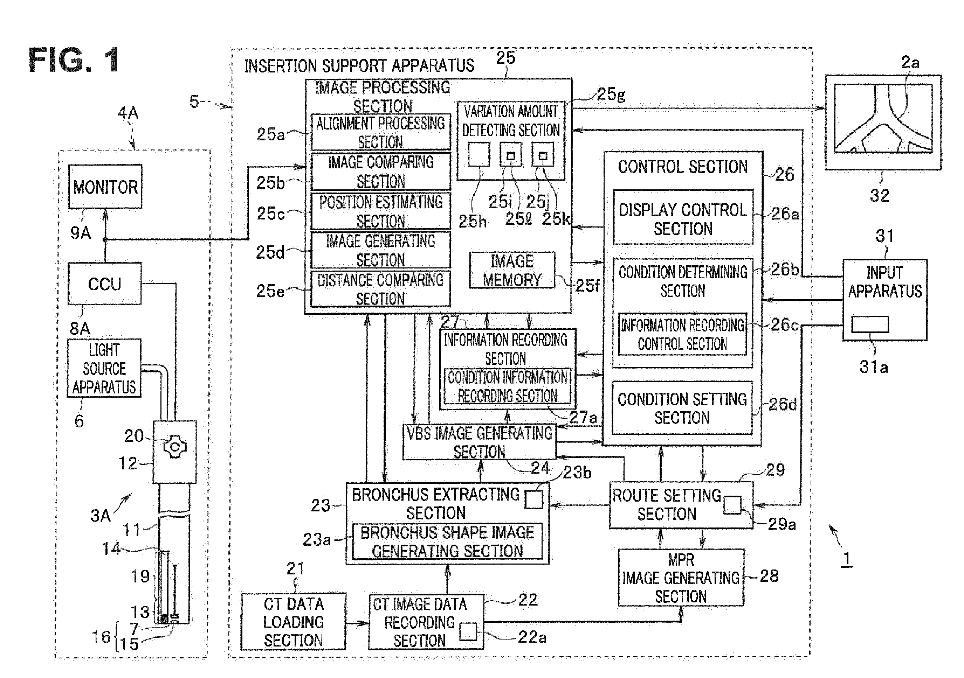

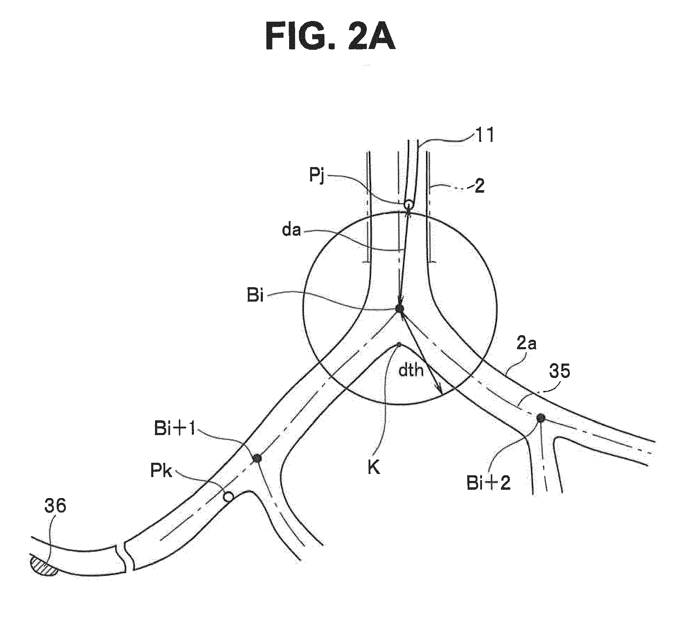

As illustrated in Fig. 1, an endoscope system 1 according to a first embodiment of the present invention mainly includes an endoscope apparatus 4A including an endoscope 3A to be inserted into a bronchus 2 (Fig. 2A), which is a predetermined luminal organ of a patient, which is a subject to be examined, and an insertion support apparatus 5 for providing support for insertion of the endoscope 3A, the insertion support apparatus 5 being used together with the endoscope apparatus 4A.

The endoscope apparatus 4A includes the endoscope 3A, a light source apparatus 6 that supplies illuminating light to the endoscope 3A, a camera control unit (abbreviated as "CCU") 8A, which serves as a signal processing apparatus that performs signal processing for an image pickup device 7, which is included in image pickup means incorporated in the endoscope 3A, and a monitor 9A that displays an endoscopic image generated by the CCU 8A.

The endoscope 3A includes an elongated insertion portion (or endoscope insertion portion) 11 having flexibility, and an operation portion 12 provided at a rear end of the insertion portion 11, and in a distal end portion 13 of the insertion portion 11, an illumination window and an observation window are provided. A light guide 14 that conveys illuminating light is inserted inside the insertion portion 11 and the operation portion 12, and an incident end of the light guide 14 is connected to a light source apparatus 6, and illuminating light generated by a non-illustrated light source lamp or LED in the light source apparatus 6 enters the incident end. The illuminating light conveyed by the light guide 14 exits forward from a light exit end (distal end face) attached to the illumination window.

Also, an objective lens 15, which is included in an objective optical system that forms an image of an object, is attached to the observation window, an image pickup device 7 such as a CCD is disposed at a position where the image is formed, and the objective lens 15 and the image pickup device 7 form an image pickup apparatus 16, which serves as image pickup means (or an image pickup section) for picking up an image of the inside of the bronchus 2, which is a predetermined luminal organ to which the insertion portion 11 is to be inserted.

The image pickup device 7 is connected to the CCU 8A via a signal wire inserted inside the insertion portion 11 and the operation portion 12. The CCU 8A generates an image signal of an picked-up image corresponding to an optical image formed on an image pickup surface of the image pickup device 7, via a non-illustrated image signal generating circuit inside the CCU 8A, and outputs the image signal to the monitor 9A. The monitor 9A displays an image (movie) of the image signal as an endoscopic image (also referred to as "picked-up image").

In the insertion portion 11 of the endoscope 3A, a bendable bending portion 19 is provided at a rear end of the distal end portion 13, and a surgeon performs an operation to rotate a bending operation knob 20 provided at the operation portion 12, enabling the bending portion 19 to bend in an arbitrary direction, i.e., upward/downward and leftward/rightward. Note that the bending operation knob 20 includes an upward/downward bending operation knob for bending the bending portion upward/downward, and a leftward/rightward bending operation knob for bending the bending portion leftward/rightward.

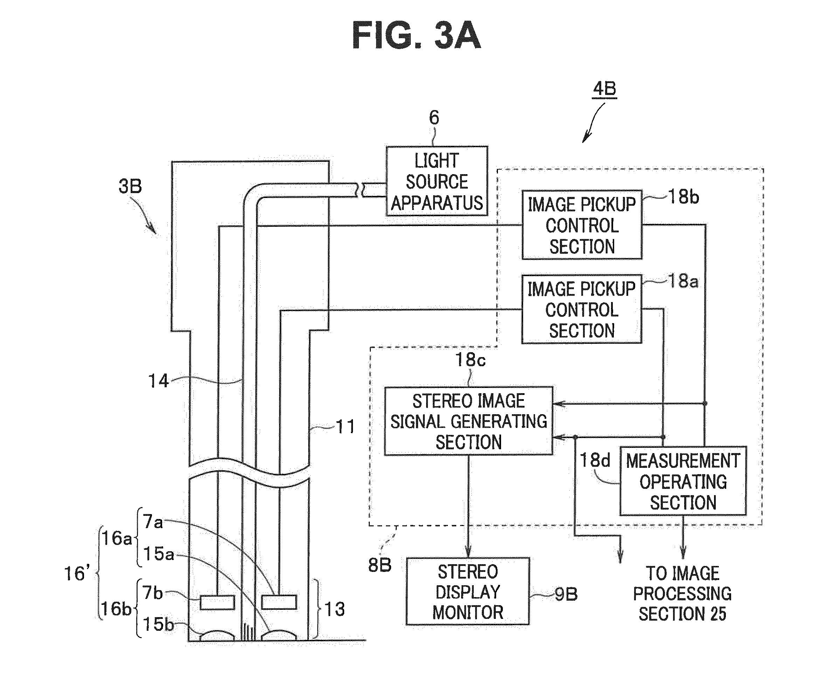

An endoscope apparatus 4B illustrated in Fig. 3A may be employed instead of the endoscope apparatus 4A illustrated in Fig. 1.

The endoscope apparatus 4B includes a stereo endoscope 3B that enables three-dimensional measurement (stereo measurement), a light source apparatus 6, a CCU 8B that performs signal processing for two image pickup devices 7a and 7b provided in the stereo endoscope 3B, and a stereo display monitor 9B that displays a stereo image signal generated by the CCU 8B.

In a distal end portion 13 of an insertion portion 11 of the stereo endoscope 3B, left and right objective lenses 15a and 15b are disposed with a predetermined space provided in a horizontal direction therebetween, and the left and right image pickup devices 7a and 7b are disposed on respective positions where respective images from the left and right objective lenses 15a and 15b are formed, whereby a stereo image pickup apparatus 16' including left and right image pickup apparatuses 16a and 16b are formed. Note that for the left and right objective lenses 15a and 15b and the left and right image pickup apparatuses 16a and 16b, those that have respective identical characteristics are used.

Also, a light guide 14 that conveys illuminating light from the light source apparatus 6 is inserted inside the insertion portion 11. A distal end of the light guide 14 is attached to an illumination window in the distal end portion 13, and the conveyed illuminating light is made to exit from the illumination window, and illuminates an object such as a diseased part inside a body cavity.

The left and right image pickup devices 7a and 7b, which pick up an image of the illuminated object, make image pickup signals resulting from photoelectric conversion be inputted to image pickup control sections 18a and 18b in the CCU 8B, and the image pickup control sections 18a and 18b generate left and right image signals and output the left and right image signals to a stereo image signal generating section 18c.

The stereo image signal generating section 18c generates an image signal for stereo display from the left and right image signals and outputs the image signal to the stereo display monitor 9B. Then, the stereo display monitor 9B displays an image signal for stereo display, and the display of the image signal for stereo display enables a user such as a surgeon to view the object stereoscopically.

Also, the left and right image signals generated by the image pickup control sections 18a and 18b are inputted to a measurement operating section 18d, and stereo measurement utilizing the principle of triangulation is performed using the left and right image signals, enabling measurement of, e.g., a distance between two points in the picked-up endoscopic image. As will be described later, for example, a bronchus diameter Da can be measured (calculated). Information such as the bronchus diameter Da calculated by the measurement operating section 18d is outputted to an image processing section 25. Note that the bronchus diameter Da measured (calculated) from the endoscopic image is not an average inner diameter of the bronchus 2, but a value of an inner diameter calculated from two points in the lumen. Thus, in the vicinity of a divergence region in which the bronchus 2 diverges, a bronchus diameter Da that is larger than an actual inner diameter of the bronchus may be measured (calculated). In Fig. 3A, a video signal generated by the image pickup control section 18a (or 18b) is also outputted to the image processing section 25.

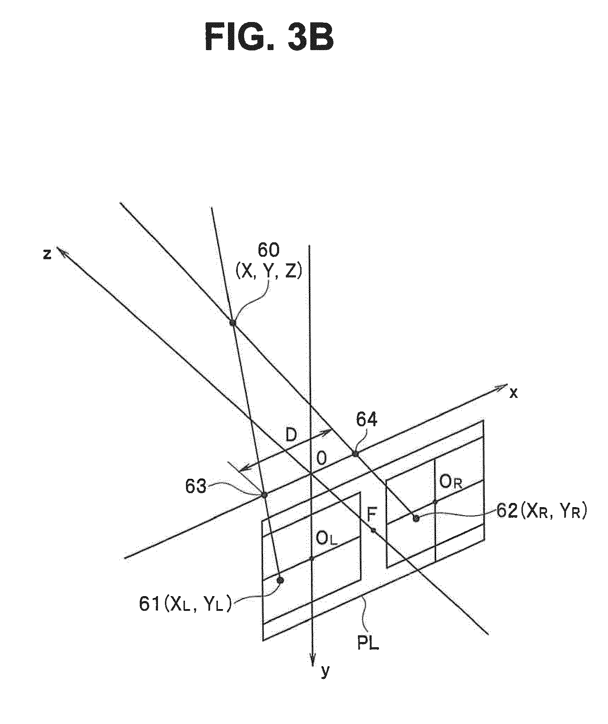

Next, a method of obtaining three-dimensional coordinates of a point (position) to be measured by stereo measurement will be described with reference to Fig. 3B. Three-dimensional coordinates (X, Y, Z) of a measurement point 60 in each of images formed on respective image pickup surfaces of the image pickup devices 7a and 7b using the left and right objective lens 15a, 15b are calculated according to the following Expressions (1) to (3) by means of the triangulation method. Here, ($X_L$, $Y_L$) and ($X_R$, $Y_R$) are respective two-dimensional coordinates of measurement points 61 and 62 on left and right images subjected to distortion correction, D is a distance between optical centers 63 and 64 of the left and right objective lenses 15a and 15b, F is a focal length, and t = D / ($X_L$ - $X_R$). Then, the following relational expression holds.

$$X = t \times X_R + D/2 \ldots (1)$$

$$Y = t \times Y_R \ldots (2)$$

$$Z = t \times F \ldots (3)$$

As a result of determination of the two-dimensional coordinate measurement points 61 and 62 in the images corresponding to the measurement point 60 as described above, three-dimensional coordinates of the measurement point 60 can be obtained using the distance D and the focal length F as parameters.

Obtainment of three-dimensional coordinates of several points enables various kinds of measurement of, e.g., a distance between two points of these points, a distance between a line connecting two points and one point from these points, area, depth, surface shape, and also enables obtainment of a distance (object distance) from the optical center 63 of the left objective lens 15a or the optical center 64 of the right objective lens 15b to an object. In order to perform the above-described stereo measurement, optical data indicating the characteristics of the distal end portion 13 and the objective lenses 15a and 15b of the endoscope 3B are used. Note that in Fig. 3B, PL denotes a plane including both of the two image pickup surfaces and $O_L$ and OR denote respective centers of the right image pickup surface (on respective optical axes of the objective lenses 15a and 15b, which are not indicated in Fig. 3B).

Examples of a method of performing an operation to calculate three-dimensional coordinates from a stereo image include the method indicated in Japanese Patent Application Laid-Open Publication No. 2011-027911.

In the present embodiment, when a bronchus diameter Da is measured, which will be described later, a distance between two points designated by designating the points 61 and 62 corresponding to one measurement point 60 of the bronchus diameter and the other measurement point of the bronchus diameter on the image pickup surface in Fig. 3B is calculated.

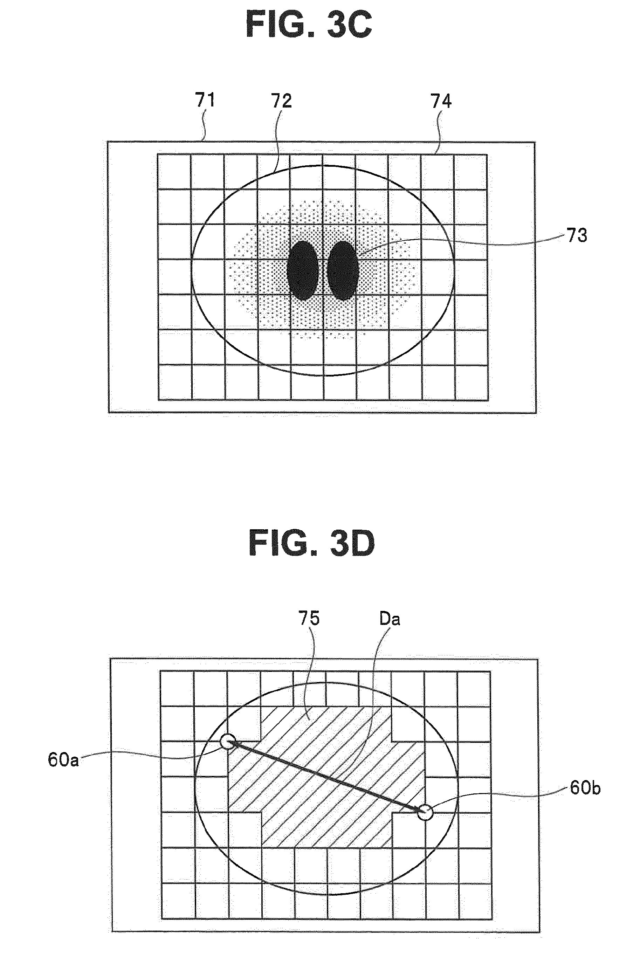

This method will be described with reference to Figs. 3C and 3D. A display screen 71 of the monitor 9B shows a bronchus 72 and a next bronchus diverging part 73 on the peripheral side of the bronchus 72 in an endoscopic image. The area of the screen 71 is segmented into blocks indicated by a mesh 74, and an area including respective blocks each having an average luminance that is equal to or below a predetermined value is extracted. Fig. 3D indicates a detection block 75 extracted as described above, by shading.

Then, two two-dimensional coordinate points that provide a largest diameter in the detection block 75 are determined as those for a bronchus diameter Da, and the two points are set as a measurement point 60a and a measurement point 60b. Note that Fig. 3D indicates one of a left screen and a right screen, and on the other screen, the measurement points 60a and 60b are set as described above. In general, when a luminal organ is observed via an endoscope, the image becomes darker as the endoscope goes deeper, and thus, measurement points can be set by the method described above. For setting measurement points 60a and 60b, a direction in which a distance between the measurement points 60a and 60b becomes largest may be designated.

The above operation is performed for each of the left screen and the right screen forming a stereo image to obtain the two-dimensional measurement point 60a and the two-dimensional measurement point 60b on each of the left screen and the right screen. Then, where the operation is performed with the point corresponding to the measurement point 60a on the left screen as the two-dimensional point 61 in Fig. 3B and the point corresponding to the measurement point 60a on the right screen as the two-dimensional point 62, a (three-dimensional coordinate) position of the measurement point 60 can be found. An operation similar to the above is performed for the point corresponding to measurement point 60b on each of the left screen and the right screen, whereby three-dimensional coordinates of each of the measurement points 60 at opposite ends of the bronchus diameter can be obtained, enabling calculation of a bronchus diameter Da (in a measurement direction connecting the two points) from the distance between the two points.

The above operation is performed each time the endoscopic image is updated, enabling monitoring of variation in the bronchus diameter Da in the measurement direction, which is calculated from the endoscopic image.

Also, stereo measurement may be performed as described below using the endoscope 3A including the monocular (single) image pickup apparatus 16 in Fig. 1 instead of the stereo endoscope 3B including the stereo image pickup apparatus 16' including the paired left and right image pickup apparatuses 16a and 16b, which is illustrated in Fig. 3A.

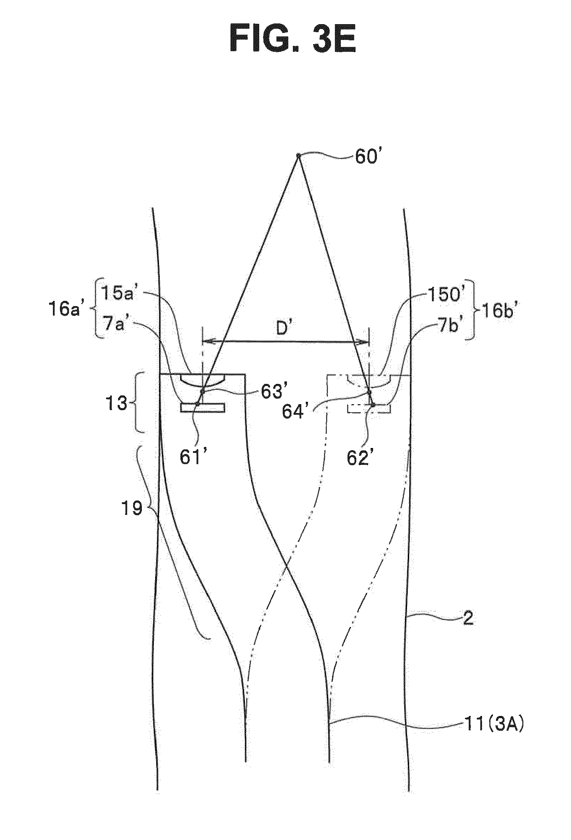

Where the endoscope 3A is inserted into a bronchus 2 as illustrated in Fig. 3E, it is possible that: a surgeon bends the bending portion 19 on the distal end of the insertion portion 11 to the left and right to achieve a state that is substantially equivalent to a state in which images are picked up by the left and right image pickup apparatuses in Fig. 3B, in order to calculate a bronchus diameter by means of stereo measurement.

For example, the distal end of the insertion portion 11 with the bending portion 19 not bent is set in the vicinity of a central line of the bronchus 2, and the surgeon bends the bending portion 19, for example, to the left to bring the distal end of the insertion portion 11 into contact with an inner wall on the left side of the bronchus 2, thereby setting the distal end of the insertion portion 11 at a first image pickup position 16a' corresponding to that of a case where an image is picked up by the left image pickup apparatus 16a in Fig. 3B. Reference numerals 15a' and 7a' denote an objective lens 15 and an image pickup device 7 at the first image pickup position 16a', respectively.

After pickup of an image at the first image pickup position 16a', the surgeon bends the bending portion 19 to the right to bring the distal end into contact with an inner wall on the right side of the bronchus 2 as indicated in the alternate long and two short dashes lines in Fig. 3E, thereby setting the distal end at a second image pickup position 16b' corresponding to that of a case where an image is picked up by the right image pickup apparatus 16b in Fig. 3B. Reference numerals 15b' and 7b' denote an objective lens 15 and an image pickup device 7 at the second image pickup position 16b', respectively. An image is picked up at the second image pickup position 16b'.

Information such as leftward/rightward movement amounts of the distal end portion 13 when the bending portion 19 is bent to the left and the right, respectively, by operating the bending operation knob 20, a focal length of the objective lens 15 of the image pickup apparatus 16, pixels counts in horizontal and vertical directions of the image pickup device 7 and a pixel pitch is obtained in advance and stored in, e.g., the information recording section 27.

In this case, optical centers 63' and 64' in Fig. 3E corresponding to the left and right optical centers 63 and 64 in Fig. 3B and a distance D' corresponding to the distance D between the left and right optical centers can be calculated from, e.g., a bending angle of the bending portion 19 (or an operation amount of the bending operation knob 20). Also, the three-dimensional position of the measurement point 60' can be calculated from information on the measurement points 61' and 62' on the image pickup devices 7a' and 7b' for the measurement point 60' corresponding to the case of the measurement point 60 in Fig. 3B. Furthermore, a bronchus diameter can be calculated by designating each of two points that are one position and the other position of a bronchus diameter as a measurement point 60'. As described above, a bronchus diameter may be calculated using the endoscope 3A in Fig. 1. Note that although the description has been provided in terms of a case where the bending portion is bent leftward and rightward, if the bending portion is bent in other directions, a bronchus diameter can also be calculated along the other directions.

As illustrated in Fig. 1, the insertion support apparatus 5 includes a CT (computed tomography) data loading section 21 that loads CT data, which is three-dimensional image information on a patient to be subjected to an examination using the endoscope 3A or 3B, the three-dimensional image information being generated by known CT, via a portable recording medium such as a DVD, a Blu-ray disc or a flash memory, and a CT image data recording section 22, which serves as image recording means for recording the CT data loaded by the CT data loading section 21.

Note that the CT image data recording section 22 may store CT data (which is three-dimensional image information on a patient that is a subject) generated by means of CT, via, e.g., a communication channel or the Internet. The CT image data recording section 22 can be provided by, e.g., a hard disk apparatus, a flash memory or a DVD.

Also, the CT image data recording section 22, which provides the image recording means, includes an associated image information recording section 22a that records associated image information in which CT image data resulting from image data being separated from the CT data and three-dimensional position data using a first coordinate system (CT coordinate system) resulting from position information being separated from the CT data, the first coordinate system corresponding to the CT image data, are associated with each other.

Also, the insertion support apparatus 5 includes a bronchus extracting section 23 including, e.g., a luminal organ extracting circuit, which serves as luminal organ extracting means for extracting three-dimensional image data on a bronchus 2, which is a predetermined luminal organ, from the CT image data in the CT image data recording section 22, and a central processing unit (abbreviated as "CPU").

The bronchus extracting section 23 generates three-dimensional shape information (shape data) indicating a hollow shape of the bronchus 2 and three-dimensional shape image information (image data) from the extracted three-dimensional data (more specifically, three-dimensional volume data) on the bronchus 2. In other words, the bronchus extracting section 23 includes a bronchus shape image generating section 23a, which serves as bronchus shape image generating means for generating a bronchus shape image 2a, which is an image of a three-dimensional hollow bronchus shape from the extracted three-dimensional data on the bronchus 2.

Also, when the bronchus extracting section 23 extracts the three-dimensional data on the bronchus 2, the bronchus extracting section 23 extracts the three-dimensional data in association with the three-dimensional position data on the first coordinate system (or CT coordinate system) corresponding to the three-dimensional data. The bronchus extracting section 23 includes an associated information recording section 23b including, e.g., a memory that records associated information in which the three-dimensional shape data (that is, the bronchus shape data) and the three-dimensional position data on the bronchus 2 are associated with each other.

Also, the insertion support apparatus 5 includes a VBS image generating section 24, which serves as virtual endoscopic image generating means for generating a virtual endoscopic image (also referred to as "VBS image"), which is a virtual endoscopic image corresponding to an endoscopic image generated by image pickup by the image pickup apparatus(es) 16 or 16a and 16b provided in the distal end portion 13 of the insertion portion 11 in the endoscope 3A or 3B. The below description will be provided for the case of the endoscope 3A if either endoscopes 3A or 3B can be employed.

Characteristics information including that of an image formation system in the image pickup apparatus 16 in the distal end portion 13 of the endoscope 3A (e.g., the focal length of the objective lens 15, and the count and the size of pixels in the image pickup device 7) is inputted to the VBS image generating section 24, for example, from an input apparatus 31 via a control section 26. Note that the characteristics information on the image pickup apparatus 16 may be inputted to the VBS image generating section 24 from the input apparatus 31, not via the control section 26.

The VBS image generating section 24 includes, e.g., an image generation circuit that, based on information on a three-dimensional position of the image pickup apparatus 16 disposed inside the distal end portion 13 of the endoscope 3A actually inserted inside the bronchus 2 (which can also be regarded as a three-dimensional position of the distal end of the insertion portion 11), the characteristics information for forming an image of an object in a bronchus 2 by the image pickup apparatus 16 and the bronchus shape data, generates a VBS image that virtually draws an endoscopic image that would be obtained by endoscopically picking up an image of the inside of the bronchus 2 with the three-dimensional position (also simply referred to as "position") as a viewpoint position, or a CPU. Note that if an axis direction of the distal end (substantially corresponding to an optical axis direction of the image pickup apparatus 16) is varied while the viewpoint position remains the same, the VBS image generating section 24 can generate a VBS image corresponding to the variation.

Therefore, if, for example, a position of the distal end of the insertion portion 11 and the (axis) direction of the distal end are designated on the CT coordinate system, the VBS image generating section 24 generates a VBS image corresponding to the designated position and direction.

Also, the insertion support apparatus 5 includes: an image processing section 25 including, e.g., a CPU or an image processing circuit, the image processing section 25 performing alignment between an endoscopic image inputted from the CCU 8A and a VBS image from the VBS image generating section 24 by means of image matching; a control section 26 including, e.g., a CPU, the control section 26 serving as control means for controlling, e.g., the image processing section 25; and an information recording section 27 including, e.g., a memory, the information recording section 27 serving as information recording means for recording information on, e.g., a VBS image for providing insertion support under the control of the control section 26 as candidate information or position and image information.

Also, the insertion support apparatus 5 includes: an MPR image generating section 28 that based on the CT image data recorded in the CT image data recording section 22, generates a CT tomographic image (referred to as "MPR image") as a multi-planar reconstruction image; and a route setting section 29, which serves as route setting means including. e.g., a pointing device such as a mouse, the route setting section 29 generating a route setting screen, which serves as an insertion route setting screen including the MPR image generated by the MPR image generating section 28, and setting a route for inserting the endoscope 3A to the target site side inside the bronchus 2.

For example, if a target site 36 is designated from the CT image data as illustrated in Fig. 2A, the route setting section 29 has a function of a route data generating section 29a such as a route data generating circuit that generates route data from an insertion start position (of the insertion portion 11) in the bronchus 2 to a target position, which is the vicinity of the target site 36, from the CT image data and the bronchus shape image 2a.

Also, the endoscope system 1 includes the input apparatus 31 including, e.g., a keyboard and/or a pointing device for inputting setting information to the route setting section 29. Also, a surgeon can input parameters and data for performing image processing to the image processing section 25 via the input apparatus 31, and select, or provide an instruction for, control operation of the control section 26.

Also, if the surgeon sets a route, the route setting section 29 sends information on the set route to the VBS image generating section 24, the MPR image generating section 28 and the control section 26. The VBS image generating section 24 and the MPR image generating section 28 generate a VBS image and an MPR image along the route, respectively, and the control section 26 controls operation of the respective sections according to the route.

The endoscopic image generated by the CCU 8A (also referred to as "actual image" or simply referred to as "image") and the VBS image generated by the VBS image generating section 24 are inputted to the image processing section 25. Also, the bronchus shape image 2a generated by the bronchus shape image generating section 23a is also inputted to the image processing section 25.

In the present embodiment, since no sensor that detects a position of the distal end of the insertion portion 11 is incorporated in the distal end portion 13 of the insertion portion 11 in which the image pickup apparatus 16 is disposed, a three-dimensional position (also simply referred to as "position") of the distal end of the insertion portion 11 is estimated (or calculated) by means of image matching in the alignment processing section 25a of the image processing section 25.

If a three-dimensional position (known position) or a position in the vicinity thereof that can be specified on the CT coordinate system from the bronchus shape image 2a, such as an entrance or a carina K (see Fig. 2A) of the bronchus 2, is set in advance as a moving image matching start position, the VBS image generating section generates a VBS image based on the position information. The alignment processing section 25a in the image processing section 25 sets the distal end of the insertion portion 11 at the three-dimensional position (known position) or the position in the vicinity thereof that can be specified by the CT coordinate system (first coordinate system) from the bronchus shape image 2a, such as the entrance or the carina of the bronchus 2, enabling estimation (or calculation) of the position of the distal end of the insertion portion 11 on the CT coordinate system.

Then, the surgeon inserts the distal end of the insertion 11 so that the endoscopic image looks the same as the VBS image. As a result of the alignment descried above, the alignment processing section 25a in the image processing section 25 compares the endoscopic image and the VBS image and starts image matching so that the endoscopic image and the VBS image are matched within a condition in which a comparison result is set (error margin that ensures a predetermined accuracy).

Thus, the image processing section 25 includes an image comparing section 25b including, e.g., an image comparison circuit, the image comparing section 25b serving as image comparing means for comparing the endoscopic image and the VBS image, and the alignment processing section 25a performs processing for alignment by means of image matching utilizing the image comparison by the image comparing section 25b.

As a result of performing the above-described alignment, the alignment processing section 25a in the image processing section 25 enables the position of the distal end of the insertion portion 11 and the axis direction of the distal end (viewpoint direction or sight line direction of the image pickup apparatus 16) to be specified by information indicating coordinates of the position and the axis direction (also referred to as "posture") on the CT coordinate system (first coordinate system).

After the alignment described above, using the information subjected to the alignment, a subsequent position of the distal end of the insertion portion 11 can be obtained as information associated with a position on the CT coordinate system (first coordinate system) based on a result of the image comparison by the image comparing section 25b. In other words, the image processing section 25 includes a position estimating section 25c that obtains the position of the distal end of the insertion portion 11 by means of estimation, as position information obtaining means for obtaining the position (information) of the distal end of the insertion portion 11. The position estimating section 25c also obtains the position of the distal end of the insertion portion 11 based on the result of the image comparison by the image comparing section 25b. For a further description, the image processing section 25 estimates a moved position of the distal end of the insertion portion 11 on the CT coordinate system after alignment by the alignment processing section 25a, from a result of comparison between the endoscopic image and the VBS image in an operation to insert the insertion portion 11 to the deep part side (peripheral side) of a bronchus 2.

In other words, along with an operation to move the distal end of the insertion portion 11 substantially along a center line 35 from a position where alignment processing was performed, (in order to insert the distal end), the image pickup apparatus 16 is moved, and thus, the endoscopic image varies.

In this case, the position estimating section 25c selects a VBS image that matches best the current endoscopic image by means of image processing, using VBS images (outputted from the VBS image generating section 24) where the distal end of the insertion portion 11 is moved on a route substantially along the center line 35, and calculates (estimates) a three-dimensional position corresponding to the selected VBS image as the position of the distal end of the insertion portion 11. As described above, the position estimating section 25c also calculates (estimates) a position of the distal end of the insertion portion 11 as well as a posture thereof (the axis direction or longitudinal direction of a part around the distal end of the insertion portion 11).

Since the distal end of the insertion portion 11 may be moved to a position deviating from the center line 35, it is possible that the VBS image generating section 24 generates a VBS image for a position decentering by a relevant amount from the center line 35, and outputs the generated VBS image to the alignment processing section 25a. Consequently, a range of position estimation by means of image matching can be expanded.

Also, a movement amount and a moved position of the distal end of the insertion portion 11 are calculated (estimated) from an amount of difference between two positions estimated by the position estimating section 25c. Also, the position estimating section 25c also can calculate (estimate) a distance between an estimated position and a specific position such as a diverging point in a feature region of a bronchus 2 (position that can be specified on the CT coordinate system).

Thus, the position estimating section 25c has a function of a distance calculating section, which serves as distance calculating means for calculating a distance from a position of the distal end of the insertion portion 11 estimated by the position estimating section 25c to a feature region such as a divergence region that diverges in the bronchus 2, which is a predetermined luminal organ. As described above, the image processing section 25 has a function of the position estimating section 25c, which serves as position information obtaining means for obtaining information on a position of the distal end of the insertion portion 11 by means of estimation. In this case, the alignment processing section 25a may be defined as a configuration having the function of the position estimating section 25c.

Note that in the present description, the distal end of the insertion portion 11 means the same as a distal end of the endoscope 3A.

Also, the image processing section 25 generates an image to be displayed on a monitor 32, which serves as image display means, under the control of, e.g., a display control section 26a in the control section 26, the display control section 26a controlling display.

Under the control of the display control section 26a, the image processing section 25 normally outputs an image signal (video signal) of a bronchus shape image 2a generated by the bronchus shape image generating section 23a to the monitor 32. Then, as illustrated in Fig. 1, the bronchus shape image 2a is displayed on the monitor 32, for example, as a two-dimensional tomographic image taken in a cross section along a direction passing through a center of a lumen. Note that the present invention is not limited to the case where the bronchus shape image 2a is displayed as a two-dimensional tomographic image, and the bronchus shape image 2a may be displayed as a three-dimensional image. If the bronchus shape image 2a is displayed as a three-dimensional image, for example, the bronchus shape image 2a may be displayed, for example, as a projection view provided by parallel projection or a perspective view so that the inside of the lumen can be seen.

Also, as illustrated in Fig. 2A, in the bronchus shape image 2a displayed on the monitor 32, a center line 35 extending through a center of the lumen of the bronchus 2 is also displayed.

Note that although the center line 35 is generated by, for example, the bronchus shape image generating section 23a, the center line 35 may be generated by the image processing section 25. Also, the image processing section 25 has a function of an image generating section 25d that generates, e.g., an image in which the position of the distal end of the insertion portion 11 estimated by the position estimating section 25c is superimposed on the bronchus shape image 2a together with the center line 35.

When a user such as a surgeon inserts the insertion portion 11 into a bronchus 2 from the distal end thereof, the center line 35 and the position of the distal end of the insertion portion 11 are displayed on a bronchus shape image 2a indicating a three-dimensional shape of the bronchus 2, and an operation to insert the insertion portion 11 can be facilitated by referring to the display. Also, an operation to insert the insertion portion 11 along the center line 35 is performed, enabling estimation of the position of the distal end of the insertion portion 11 by image matching to be performed in a short period of time.

Also, the image processing section 25 includes a distance comparing section 25e, which serves as distance comparing means for comparing a distance from the position of the distal end of the insertion portion 11 estimated by the position estimating section 25c to a feature region inside a bronchus 2, which is a predetermined luminal organ, with a set distance.

Note that instead of the image processing section 25 having a configuration including the distance comparing section 25e, the control section 26 may be configured to include a distance comparing section 25e, which serves as the distance comparing means. Although it has been described above that the position estimating section 25c in the image processing section 25 calculates (estimates) a distance, the distance comparing section 25e may perform distance calculation (estimation) and distance comparison.

Furthermore, in the present embodiment, the image processing section 25 includes a variation amount detecting section 25 g, which serves as variation amount detecting means for detecting an amount of variation of a feature part in an endoscopic image (also simply referred to as "image") picked up by the image pickup apparatus 16. The variation amount detecting section 25g includes a bronchus diameter variation amount detecting section 25h that detects an amount of variation in bronchus diameter (inner diameter of a bronchus 2) as a feature part, a brightness variation amount detecting section 25i that detects an amount of variation in brightness of a divergence region as a feature part, and a shape variation amount detecting section 25j that detects an amount of variation in shape of a divergence region.

Also, the shape variation amount detecting section 25j includes a spur variation amount detecting section 25k that detects an amount of variation in length or angle of a spur (a diverging point or a divergence boundary) in which a lumen of a bronchus 2 is divided (diverges), and the brightness variation amount detecting section 25i has a function of a later-described poor visibility detecting section 251. The present invention is not limited to the case where the brightness variation amount detecting section 25i has the function of the poor visibility detecting section 251.

Note that the control section 26 may correct route data generated (before insertion of the insertion portion 11 of the endoscope 3A) by the route data generating section 29a, according to the position of the distal end of the insertion portion 11 estimated by the position estimating section 25c.

Also, the control section 26 has a function of the condition determining section 26b that determines whether or not a result of comparison by the distance comparing section 25e and a result of detection by the variation amount detecting section 25g meet predetermined conditions for recording.

If the condition determining section 26b in the control section 26 determines that the predetermined conditions are met, the condition determining section 26b makes information associating information on a position and a posture of the distal end of the insertion portion 11 estimated by the position estimating section 25c when it is determined that the predetermined condition are met, and a VBS image corresponding to the information on the position and the posture with each other (as position and image information or candidate information to be presented at the time of realignment) be recorded in the information recording section 27.

Thus, the information recording section 27 has a function of information recording means for recording position and image information (also simply referred to as "information"), which is used as candidate information associating information on a position and a posture of the distal end of the insertion portion 11 and a VBS image corresponding to the information on the position and the posture with each other, based on a result of comparison by the distance comparing section 25e and a result of detection by the variation amount detecting section 25g.

Also, the condition determining section 26b in the control section 26 has a function of an information recording control section 26c that performs control to record the position and the image information in the information recording section 27.

Also, for example, the display control section 26 in the control section 26 performs control to, in cases where an instruction signal for realignment is inputted from the input apparatus 31, for example, a case where a surgeon considers an accuracy of a current estimated position of the distal end of the insertion portion 11 as low, read the information recorded in the information recording section 27 and display the information on the monitor 32 via the image processing section 25 as candidate information.

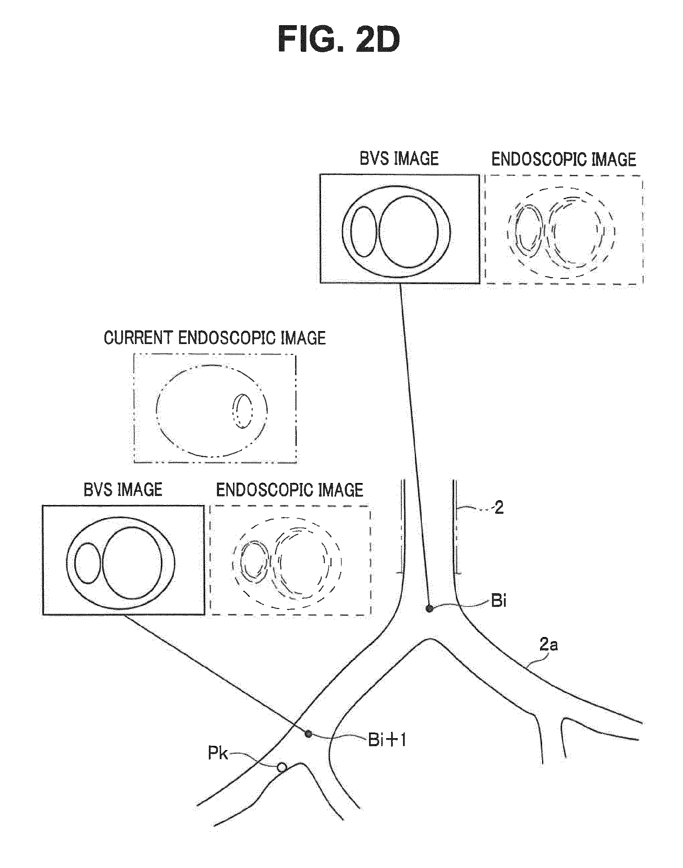

In this case, the image processing section 25 includes the image generating section 25d that generates an image indicating the candidate information read from the information recording section 27 superimposed on a bronchus shape image 2a. More specifically, a position and a posture of the distal end of the insertion portion 11 and a VBS image corresponding to the position and the posture are superimposed and displayed on a bronchus shape image 2a. Note that, as will be described later, Fig. 2D illustrates a state in which positions of the distal end of the insertion portion 11 are displayed at respective positions corresponding to the positions in a bronchus shape image 2a displayed on the monitor 32 and respective VBS images corresponding to the positions are associated (by lines), superimposed and displayed in the bronchus shape image 2a.

The surgeon can perform realignment with reference to the candidate information, and the alignment processing section 25a or the position estimating section 25b can obtain information on a position and a posture of the distal end of the insertion portion 11 in such a manner the information is associated with a coordinate system for the bronchus 2. Then, as a result of the realignment, the position estimating section 25b ensures a predetermined accuracy, enabling an operation to insert the distal end of the insertion portion 11 again from the position where realignment was performed to the deep part side of the bronchus 2.

In the present embodiment, as described above, if a result of determination is that a result of comparison by the distance comparing section 25e meets a first condition and a result of detection by the variation amount detecting section 25g meets a second condition (meet the predetermined conditions including the first condition and the second condition), the information recording control section 26c or the condition determining section 26b makes information including an (estimated) position and posture of the distal end of the insertion portion 11 when such result of determination was obtained, and a VBS image corresponding to the position and the posture be recorded in the information recording section 27 as candidate information. Note that information forming candidate information including at least a position from among a position and a posture of the distal end may be recorded in the information recording section 27.

In the present embodiment, as result of information being recorded when a plurality of conditions that are different from each other are met, when realignment is performed, a proper amount (or proper number) of candidate information (pieces) can be displayed (or presented) on the monitor 32, which serves as display means (or a display apparatus).

In the present embodiment, an amount of variation of a feature part such as a bronchus diameter of a bronchus 2, which is a predetermined luminal organ, in an endoscopic image picked up by the image pickup apparatus 16 is detected by the variation amount detecting section 25g, and based on at least a result of the detection by the variation amount detecting section 25g, (information) on a position and a posture of the distal end of the insertion portion 11 when the result of the detection was provided, and information including a VBS image corresponding to the position and the posture are recorded in the information recording section 27 (as candidate information to be presented when realignment is performed).

A user such as a surgeon can easily grasp conditions or situations for recording information because the user performs an operation to insert the insertion portion 11 while observing an endoscopic image picked up by the image pickup apparatus 16. Also, candidate information presented when realignment is performed can be related to a feature part in an endoscopic image, a variation amount of which sensitively varies in response to movement of the position of the distal end of the insertion portion 11, facilitating alignment by means of image comparison.

Note that information recorded in the information recording section 27 includes a position and a posture of the distal end of the insertion portion 11 and a corresponding VBS image, but may include an endoscopic image corresponding to the position and posture information.

Also, the image processing section 25 includes an image memory 26f that when image matching is performed by comparing an endoscopic image and a VBS image with each other, temporarily stores the endoscopic image and the VBS image, or is used as a work area for image processing. Note that an image memory 25f may be provided outside the image processing section 25.

Also, in the present embodiment, for example, the input apparatus 31 may be configured so as to include a designation section 31a that selectively designates (or sets) a first condition relating to the distal end of the insertion portion 11 and a feature region for which the distance comparing section 25e performs comparison, and a second condition relating to an amount of variation in feature part detected by the variation amount detecting section 25g, respectively.

Also, for example, the information recording section 27 may include a condition information recording section 27a, which serves as condition information recording means for recording first condition candidate information relating to the first condition and second condition candidate information for the second condition in advance in addition to information to be used as candidate information described above. Note that a configuration in which the condition information recording section 27a is provided separately from the information recording section 27 may be employed.

Examples of the first condition candidate information include: (a) a distance da between the distal end of the insertion portion 11 and a diverging point Bi (i = 1,2, ...) on a center line 35; (b) a distance db between the distal end of the insertion portion 11 and a spur Spi(i = 1,2, ...) in which a bronchus 2 diverges; (c) a distance dc between the distal end of the insertion portion 11 and a center line 35 or a bronchus wall; and (d) a distance dd between the distal end of the insertion portion 11 and a region set in advance. Furthermore, it is also possible that a user such as a surgeon selectively designates any of first condition candidate information (a) to (d) via the designation section 31a so that the user can use such first condition candidate information as (information for) the first condition. In addition to the above, examples of the first condition candidate information may include: (e) a distance from the distal end of the insertion portion 11 to a target site and (f) a distance from the distal end of the insertion portion 11 to an insertion start position.

Examples of the second condition candidate information include: (a) variation in bronchus diameter Da; (b) variation in brightness of an image (endoscopic image) or a display screen that displays an endoscopic image; (c) variation in shape of a divergence; (d) variation in length of a spur Sp; (e) variation in angle of a spur Sp; (f) poor visibility; (g) large displacement of an endoscopic image; and (h) change in an endoscopic image such as appearance of an object other than a bronchus. It is also possible that a user such as a surgeon selectively designates any of the second condition candidate information (a) to (h) via the designation section 31a including, e.g., a mouse and/or a keyboard, which serves as designation means so that the user can use such second condition candidate information as (information for) the second condition.

In this case, the control section 26 has a function of a condition setting section 26d that sets a first condition and a second condition in response to designation via the designation section 31 a. When the condition setting section 26d sets a first condition and a second condition, the condition setting section 26d also sets threshold value information used when the condition determining section 26b performs determination. Note that the threshold value information may also recorded in the information recording section 27 in association with the first condition candidate information.

Although in Fig. 1, for example, the image processing apparatus 25 may be formed by a CPU (central processing unit), each of the alignment processing section 25a to the variation amount detecting section 25g in the image processing section 25 may be formed using respective pieces of dedicated hardware other than a CPU. Also, the control section 26 in Fig. 1 may be formed by a CPU or may be formed using dedicated hardware other than a CPU.

The endoscope system 1 having such configuration as described above includes: the CT image data recording section 22, which serves as image recording means for recording three-dimensional image information on a subject, the three-dimensional image information being obtained in advance; the bronchus extracting section 23, which serves as luminal organ extracting means for extracting a bronchus 2, which is a predetermined luminal organ, from the three-dimensional image information; the VBS image generating section 24, which serves as virtual endoscopic image generating means for generating a virtual endoscopic image drawn like an endoscopically obtained image from a predetermined viewpoint position for information on the predetermined luminal organ extracted by the luminal organ extracting means; the image pickup apparatus 16 or 16' provided inside the endoscope 3A or 3B, the image pickup apparatus 16 or 16' serving as image pickup means for picking up an image of the inside of the predetermined luminal organ; the position estimating section 25c, which serves as position information obtaining means for obtaining information on a position of the distal end of the insertion portion 11 of the endoscope 3A inside the predetermined luminal organ as position information; the distance comparing section 25e, which serves as distance comparing means for comparing a distance from the position of the distal end of the insertion portion 11 of the endoscope 3A or 3B obtained by the position information obtaining means to a feature region in the predetermined luminal organ extracted by the luminal organ extracting means, with a set distance; the variation amount detecting section 25g, which serves as variation amount detecting means for detecting an amount of variation in a feature part in the predetermined luminal organ in an endoscopic image, which is the image picked up by the image pickup means; and the information recording section 27, which serves as information recording means for, based on a result of the comparison by the distance comparing means and a result of the detection by the variation amount detecting means, recording position and image information including the virtual endoscopic image corresponding to the information on the position of the distal end of the insertion portion of the endoscope.

Next, operation of the present embodiment will be described.

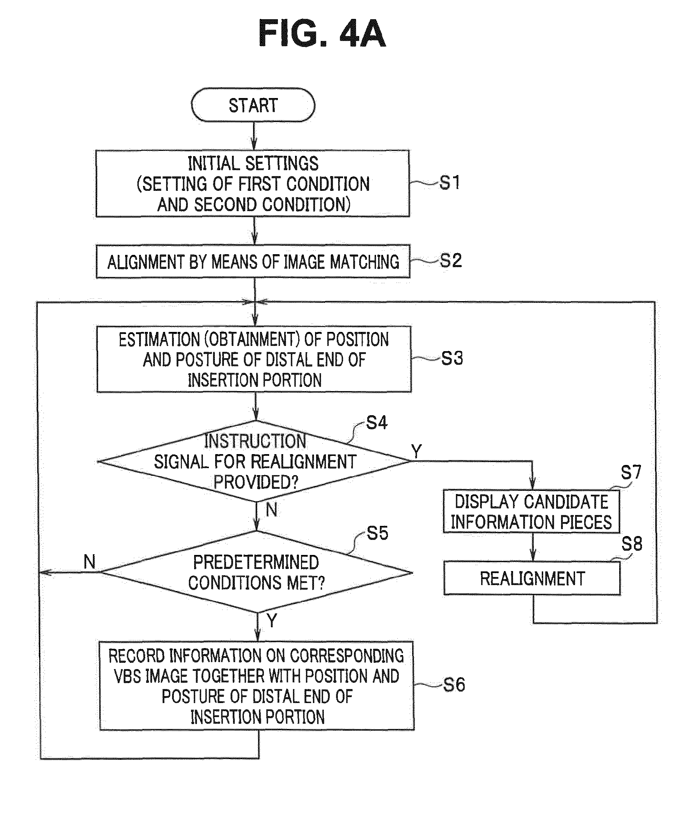

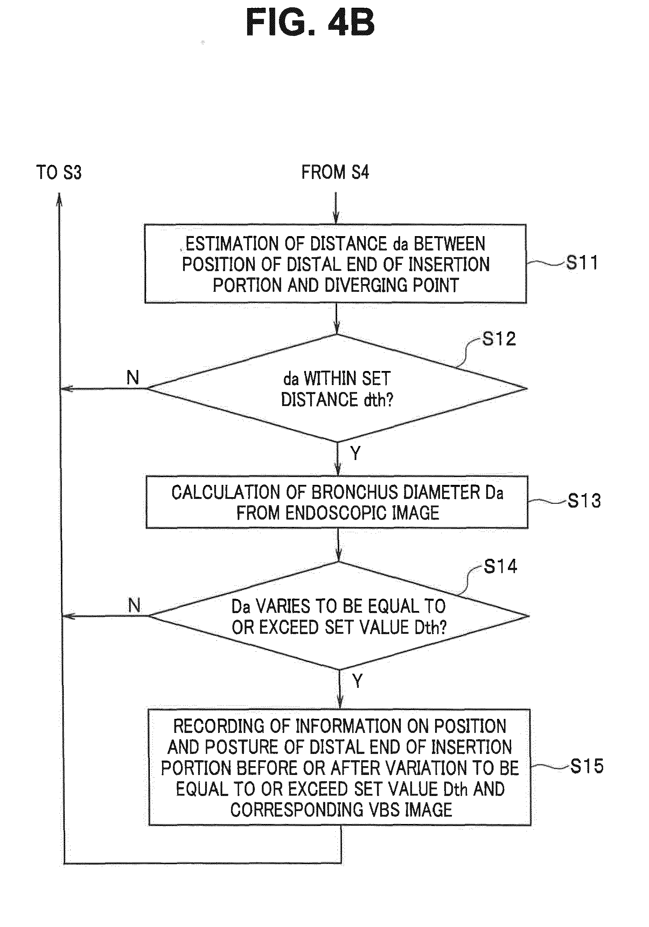

Fig. 4A illustrates typical processing in the present embodiment, and Fig. 4B illustrates a part of the processing in Fig. 4A, that is, the processing part of recording (position and image) information that can be used as candidate information, when predetermined conditions are met.

When the endoscope system 1 in Fig. 1 is powered on and the endoscope apparatus 4A (or 4B) and the insertion support apparatus 5 thereby enter an operating state, the processing in Fig. 4A starts. In first step S1 in Fig. 4A, initial setting processing is performed. As the initial setting processing, a surgeon inputs information to be used for insertion support in the present embodiment, via the input apparatus 31. In this case, the surgeon designates a first condition and a second condition via the designation section 31a. Also, the condition determining section 26b enters a state in which the condition determining section 26b makes determination according to the designated first condition and second condition.

A case where the surgeon designates a distance da between the distal end of the insertion portion 11 and a diverging point Bi at which a center line 35 splits as the first condition, and variation of a bronchus diameter Da as the second condition will be described below as case (A).

(A) Case where a distance da between (a position of) the distal end of the insertion portion 11 and a diverging point Bi and variation of a bronchus diameter Da are designated as the first condition and the second condition

As illustrated in Fig. 2A, the bronchus shape image generating section 23a generates a bronchus shape image 2a as an image of a shape of a bronchus 2 and the bronchus shape image 2a is displayed on the monitor 32 through the image processing section 25. Furthermore, as described above, a center line 35 extending through a center of a lumen of the bronchus 2 is displayed in the bronchus shape image 2a. Diverging points Bi at which the center line 35 diverges are also displayed on the monitor 32. Respective positions of the center line 35 and the diverging points Bi are known three-dimensional positions identified by the CT coordinate system.

In next step S2, the surgeon inserts the distal end of the insertion portion 11 into the bronchus 2. Here, the surgeon inserts the distal end of the insertion portion 11 so that a VBS image of, e.g., an entrance of the bronchus 2 or a carina K (see Fig. 2A) set in advance as an image matching start position and an endoscopic image provided by the image pickup apparatus 16 (or 16') look the same as each other. As a result of such alignment, the alignment processing section 25a in the image processing section 25 starts image matching so that the endoscopic image and the VBS image correspond to each other within a condition (error margin that ensures a predetermined accuracy). Note that where the image pickup apparatus 16' is used, an endoscopic image provided by either image pickup apparatus 16a or 16b in the image pickup apparatus 16' may be employed.

After the alignment processing in step S2, the surgeon inserts the distal end of the insertion portion 11 to the deeper part side of the bronchus 2 relative to the position at which the alignment was performed. When the insertion portion 11 is inserted, as illustrated in step S3, the position estimating section 25c in the image processing section 25 estimates a position and a posture of the distal end of the insertion portion 11 by means of image matching using the image comparing section 25b. If the position and the posture can be estimated by means of image matching, as illustrated in Fig. 2A, the estimated position is displayed at a corresponding position in the bronchus shape image 2a. Furthermore, the information is stored in, for example, the image memory 25f.

Also, as illustrated in next step S4, the control section 26 monitors whether or not an instruction signal for realignment is inputted by, e.g., the surgeon.

As described above, in step S2, if a travel distance from the position at which the alignment was performed is not large, the surgeon does not need to provide an instruction for realignment. Note that the image comparing section 25b in the image processing section 25 may be configured to, if the image comparing section 25b compares the endoscopic image and the VBS image and the image comparison shows that the degree of matching of the images indicates a misalignment that is equal to or exceeds a preset threshold value, in other words, if the estimation of the position of the distal end of the insertion portion 11 fails, generate an instruction signal for realignment and input the instruction signal to the control section 26. In this case, also, if a travel distance from the position at which the alignment was performed is not large, no instruction signal is inputted to the control section 26.

If no instruction signal is inputted to the control section 26, in step S5, the condition determining section 26b in the control section 26 determines whether or not a result of comparison by the distance comparing section 25e and a result of detection by the variation amount detecting section 25 g meet predetermined conditions.

In step S5, if a determination result that the predetermined conditions are not met is provided, the processing returns to step S3. On the other hand, if a determination result that the predetermined conditions are met is provided, the processing proceeds to step S6, and, for example, the condition determining section 26b performs control so as to record information on a position and a posture of the distal end of the insertion portion 11 when the determination result that the predetermined conditions are met is provided and a corresponding VBS image in information recording section 27.

Note that since the determination result that the predetermined conditions are met is a result of comparison for at least two or more timings that are mutually different in time as will be described later, information on a position and a posture of the distal end of the insertion portion 11 at one time from among the two or more times for which the comparison was performed and a VBS image corresponding to the position and the posture is recorded in the information recording section 27. After the processing in step S6, the processing returns to step S3. Consequently, if the distal end of the insertion portion 11 travels relatively farther from the position at which first alignment was performed, the accuracy of matching by the image comparing section 25b is likely to be lowered.

If the matching accuracy is lowered, an instruction signal for realignment is inputted to the control section 26, and the control section 26 detects the input of the instruction signal. Then, as illustrated in step S7, the display control section 26a in the control section 26 performs control so as to read the information recorded in the information recording section 27 as candidate information and present or display the candidate information on the monitor 32.

In next step S8, the surgeon performs realignment with reference to the candidate information displayed on the monitor 32, and then the processing returns to step S3, and the old alignment information in step S2 is updated. Note that after the processing in step S7, it is possible that the processing returns to step S2 (not to step S3) and the surgeon performs realignment with reference to the candidate information. Consequently, the processing in Fig. 4A is repeated, enabling the operation to insert the insertion portion 11 to the peripheral side (deep part side) of the bronchus 2 to be performed smoothly.

Next, the processing in steps S5 and S6 in Fig. 4A will be described in more detail with reference to Fig. 4B. Note that the description will be provided on the premise that for the processing relating to bronchus diameter in Fig. 4B, stereo measurement by the endoscope apparatus 4B, which has been described with reference to Fig. 3A, or the stereo measurement in Fig. 3E is used.

In step S4 in Fig. 4A, if no instruction signal for realignment is inputted to the control section 26, in step S 11 in Fig. 4B, the position estimating section 25c in the image processing section 25 calculates, from (information on) the position of the distal end of the insertion portion 11 obtained in step S3 and (position information on) a diverging point Bi in three-dimensional data of the bronchus 2 extracted by the bronchus extracting section 23, a distance da therebetween. Fig. 2A illustrates the bronchus 2 (and the bronchus shape image 2a thereof) and the insertion portion 11 inserted inside the bronchus 2.

As illustrated in Fig. 2A, if the distal end of the insertion portion 11 is located at a position Pj on the insertion entrance side relative to the diverging point Bi, a set distance dth, which is a threshold value distance set (in advance for the diverging point Bi), is set (with the designation of the first condition) for the distance da between the distal end of the insertion portion 11 and the diverging point Bi positioned forward relative to the position Pj.

In next step S 12, the distance comparing section 25e determines whether or not the first condition that the distance da between the calculated position Pj of the distal end of the insertion portion 11 and the diverging point Bi is within the set distance dth is met.

If a result of the determination that the first condition is not met is provided in the determination processing in step S12, the processing returns to step S3. On the other hand, if a result of the determination that the first condition is met is provided in the determination processing in step S12, in next step S 13, a bronchus diameter Da is calculated from information in the endoscopic image as described above by means of (the measurement operating section 18d in) the endoscope apparatus 4B or stereo measurement utilizing bending of the bending portion 19 of the endoscope 3A. Then, information on the calculated bronchus diameter Da is sent to the variation amount detecting section 25g in the image processing section 25.

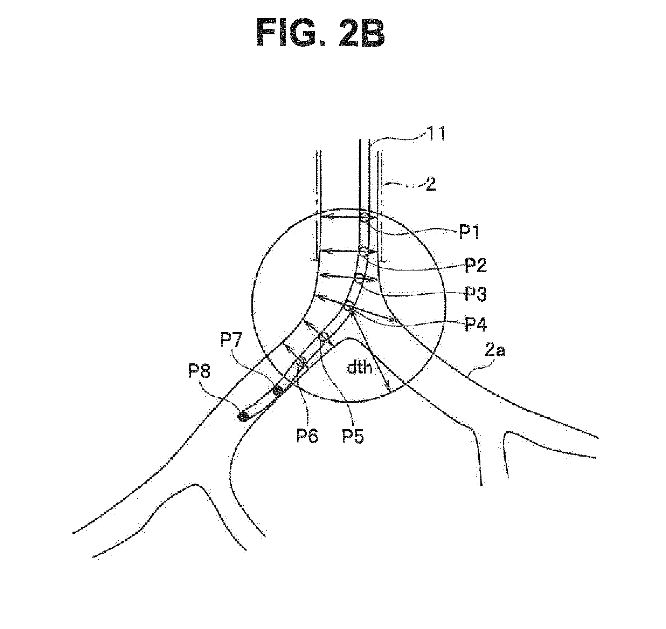

In next step S 14, (the bronchus diameter variation amount detecting section 25h in) the variation amount detecting section 25g determines whether or not the second condition that the calculated bronchus diameter Da has varied by an amount that is equal or exceeds a set value Dth, which is threshold value information set in advance for the bronchus diameter Da is met. Fig. 2B illustrates a state in which the insertion portion 11 is inserted to the peripheral side of the bronchus 2 from the position at which the distal end of the insertion portion 11 meets the first condition as illustrated in Fig. 2A.

As illustrated in Fig. 2B, the position Pj of the distal end of the insertion portion 11 is estimated and obtained, for example, at fixed time intervals by the position estimating section 25c, and the estimated position Pj moves to a current position P8 of the distal end through positions P1, P2, ..., P6 and P7. Note that the present invention is not limited to the case where the position is estimated at fixed time intervals, and the position may be estimated for, e.g., every fixed distance, every predetermined number of operations to calculate the position of the distal end of the insertion portion 11 or every predetermined number of operations to calculate the bronchus diameter.

Also, each of positions Pj (j = 1, 2, ..., 6 in Fig. 2B) indicated by white circles in Fig. 2B is a position that meets the first condition for the diverging point Bi, and each of the positions P7 and P8 indicated by black circles is a position that falls outside the first condition for the diverging point Bi. However, processing similar to that of the case of diverging point i is performed for a next diverging point Bi+1.

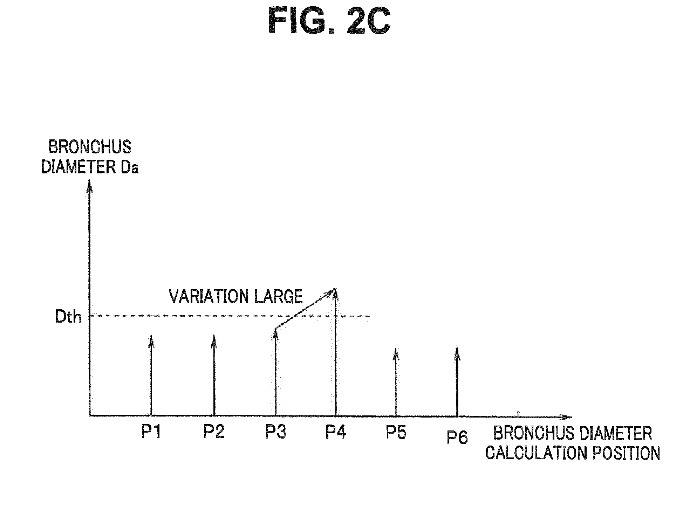

Also, Fig. 2B illustrates an overview of variation of the bronchus diameter Da calculated by the measurement operating section 18d at each of the positions Pj, and Fig. 2C illustrates the positions P1 to P6 for which the bronchus diameter was calculated in respective bronchus diameter calculations, and variation of the calculated bronchus diameter Da during travel of the distal end of the insertion portion 11 in a state in which the first condition is met. Note that information on the respective positions P1 to P6 meeting the first condition is temporarily stored in, e.g., the image memory 25f.

As illustrated in Figs. 2B and 2C, the bronchus diameter Da largely varies in the vicinity of the diverging point Bi in such a manner the bronchus diameter Da reaches a peak. In this case, when the position moves from the position P3 to the position P4, the bronchus diameter Da varies to have a large value exceeding the set value Dth from a state in which the bronchus diameter Da is smaller than the set value Dth.

Thus, the condition determining section 26b in the control section 26 makes information on the position P3 and the posture or the position P4 and the posture when the position varies from the position P3 to the position P4 and the VBS image corresponding to the position P3 and the posture or the position P4 and the posture be recorded in the information recording section 27 (as candidate information). In other words, as illustrated in step S15 in Fig. 4B, information on the position (P3 or P4) and the posture of the distal end of the insertion portion 11 before or after variation of the bronchus diameter Da by an amount that is equal to or exceeds the set value Dth and the corresponding VBS image is recorded in the information recording section 27 (as candidate information).

Note that although when the position varies from the position P4 to the position P5, the bronchus diameter Da also varies from the large value to a small value, variation from the position P1 from which the state in which the first condition is met starts is within the set value Dth, and thus, no information is recorded. As described above, instead of recording information on the position P3 and the posture or the position P4 and the posture and the VBS image corresponding to the position P3 and the posture or the position P4 and the posture in the information recording section 27 (as candidate information), information on a position and a posture between the positions P3 and P4 and the corresponding VBS image may be recorded in information recording section 27.

After recording of the information on the vicinity of the diverging point Bi in step S15 as described above, the processing returns to step S3. Then, processing similar to the above is repeated. For example, as illustrated in Fig. 2B, when the reduction position is the position P8, processing similar to that of the case of the diverging point Bi is repeated for the next diverging point Bi+1. Consequently, the insertion portion 11 is inserted to a position on the peripheral side of the bronchus 2, and when the distal end of the insertion portion 11 is inserted to the vicinity of a target site, the processing in Fig. 4A or 4B ends. As can be understood from the above description, the bronchus diameter largely varies in the vicinity of each diverging point Bi in the bronchus 2, and in the present embodiment, information is recorded for the vicinity of each diverging point Bi.

Thus, for example, if an instruction signal for realignment is inputted to the control section 26 when the insertion portion is inserted to the peripheral side relative to the diverging point Bi+1 in Fig. 2A (for example, a position Pk in Fig. 2A), the information recorded in the information recording section 27 for the vicinity of the diverging point Bi and the information recorded in the information recording section 27 for the vicinity of the diverging point Bi+1 are displayed on the monitor 32 as candidate information pieces.

Fig. 2D illustrates an example of the display of the candidate information pieces in this case. In the example of the display of the candidate information pieces in Fig. 2D, the positions of the distal end of the insertion portion 11 recorded in the information recording section 27 before the distal end reached the position Pk (the diverging points Bi and Bi+1 in Fig. 2D, and the VBS images corresponding to the respective positions are displayed so as to, for example, be connected via respective lines. Note that, e.g., a diverging point Bi-1 on the root side (insertion entrance side) relative to the diverging point Bi may be displayed in such a manner as described above.

As illustrated in Fig. 2D, the information pieces recorded respectively for the vicinities of the diverging points Bi and Bi+1 in which the lumen, which is a characteristic region in the bronchus 2, diverges, are displayed as candidate information pieces for realignment. As described above, a requisite minimum number of candidate information pieces for the vicinities of the respective diverging points that are suitable for realignment are displayed. Therefore, the surgeon can easily perform realignment smoothly in a short period of time. In other words, information to be recorded is reduced by the first condition and the second condition, enabling the amount of information that is suitable (not too much) for performing realignment to be recorded.

Note that although Fig. 2D illustrates an example of presentation of candidate images for the diverging points Bi and Bi+1, for the position Pk of the distal end of the insertion portion 11, information recorded in the information recording section 27 for an estimated position Pk of the distal end of the insertion portion 11 that is closest to the position Pk may be presented as candidate information. If such case is applied to Fig. 2D, only the information recorded in the vicinity of the diverging point Bi+1 may be presented as candidate information.

On the other hand, in the conventional examples, information to be recorded (in an information recording section 27) is not reduced by the first condition for a distance between the distal end of the insertion portion 11 and a diverging point Bi, which is a feature region, and the second condition for a variation amount of an inner diameter of a lumen, which is a feature part, in an endoscopic image (more specifically, a variation amount of a bronchus diameter) in the present embodiment, too much candidate information is displayed for realignment, requiring time until realignment is performed with proper candidate information.

Also, in the present embodiment, information is recorded when a feature part in the endoscopic image largely varies with movement of the position of the distal end of the insertion portion 11, and thus, a user such as a surgeon can easily visually grasp the condition for recording information. Therefore, the present embodiment enables an amount of information that is suitable for realignment to be recorded under the condition that can easily visually be grasped by a user.

Furthermore, in the present embodiment, when the recorded information is displayed (presented) as candidate information to perform realignment, such display (presentation) reflects the characteristic of a feature part in the endoscopic image largely varying with movement of the position of the distal end of the insertion portion 11, enabling a user to easily visually perform realignment by means of image matching.

Note that as illustrated in Fig. 2C, when the bronchus diameter Da has varied, information for the position P4 at which the bronchus diameter Da reaches a maximum (peak) on a trajectory of movement of the position of the distal end may be recorded in the information recording section 27.

As a result of the recording as described above, when the information is presented as candidate information for performing realignment, if the position of the distal end of the insertion portion 11 is moved in the vicinity of a position near the position for the candidate information, the bronchus diameter in the endoscopic image largely varies with the variation of the position of the insertion portion 11, enabling an increase in degree of variation in image comparison for realignment. Also, the position is in the vicinity of the position at which the bronchus diameter in the endoscopic image reaches a maximum and thus is a position that can easily be identified.

Also, as further indicated by the alternate long and two short dashes lines in Fig. 2D, the current endoscopic image may be displayed on the monitor 32 together with the VBS images (and the endoscopic images), which is included in candidate information read from the information recording section 27 and displayed, as a combined image in which current endoscopic image and the VBS images (and the endoscopic images) are superimposed on the bronchus shape image 2a. As a result of the current endoscopic image being displayed adjacent to candidate information as described above, alignment by means of image comparison with the candidate information can easily be performed.

Furthermore, in this case, a configuration in which an image moving section that enables the display position of a VBS image on the candidate information side to be moved so as to overlap the display position of the current endoscopic image or the display position of an endoscopic image on the candidate information side to be moved so as to overlap the display position of the current endoscopic image is provided in the image processing section 25 may be employed. Alternatively, a configuration including an image moving section that enables the display position of the current endoscopic image to be moved to the display position of a VBS image on the candidate information side or the display position of an endoscopic image on the candidate information side may be employed.

Also, although Fig. 2D illustrates an example in which candidate information for the vicinities of two diverging points Bi and Bi+1 is displayed (presented), only information recorded last may be displayed (presented) as candidate information. Where such case is applied to Fig. 2D, only the information for the vicinity of the diverging point Bi+1 is displayed (presented) as candidate information.

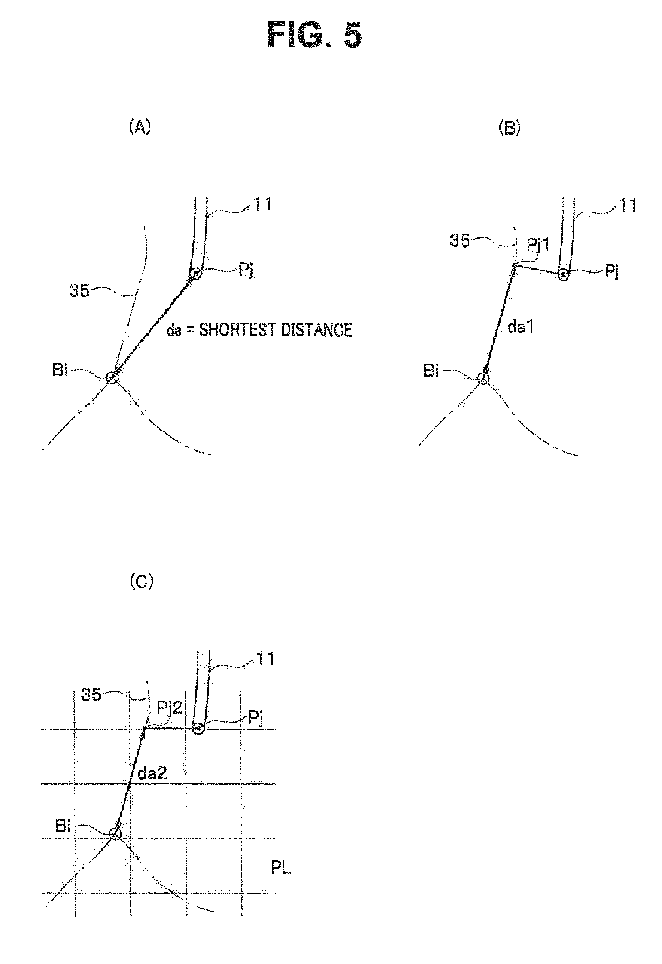

Note that when the distance da between the diverging point Bi and the position Pj of the distal end of the insertion portion 11 is calculated (measured), the calculation is performed as in any of the examples illustrated in Figs. 5(A) to 5(C).

In the example illustrated in Fig. 5(A), the distance da between the diverging point Bi and the position Pj of the distal end of the insertion portion 11 is calculated so as to be a shortest distance connecting the diverging point Bi and the position Pj. In Fig. 5(B), a position Pj 1 on the center line 35 that is a shortest distance from the position Pj of the distal end of the insertion portion 11 may be set and a distance da1 between the position Pj 1 and the diverging point Bi may be employed instead of the distance da.

Although in Fig. 5(B), the position Pj 1 on the center line 35 that is a shortest distance from the position Pj of the distal end of the insertion portion 11 is set, instead, as illustrated in Fig. 5(C), a distance da2 between a position Pj2, which is obtained by the position Pj of the distal end of the insertion portion 11 being moved onto the center line 35 on a plane parallel to a coordinate plane of a CT coordinate system corresponding to three-dimensional data of the bronchus 2, and the diverging point Bi, may be employed as the distance da.

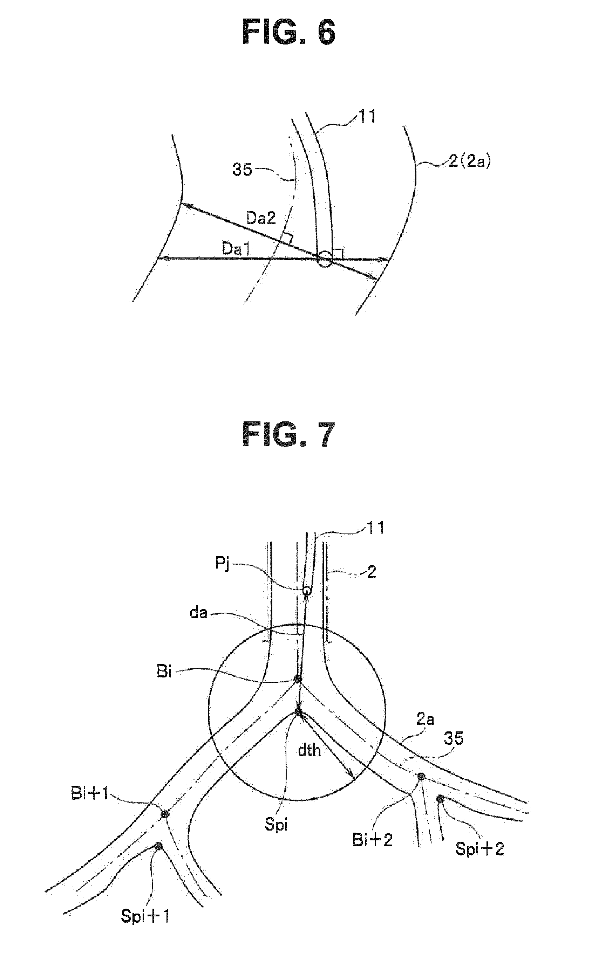

Also, when the bronchus diameter Da is calculated (measured), as illustrated in Fig. 6, a bronchus diameter Da1 calculated on a plane perpendicular to (the axis direction of) the distal end of the insertion portion 11 may be employed, or a bronchus diameter Da2 calculated along a plane perpendicular to the center line 35 through the distal end and a point on the center line 35 that is a shortest distance from the distal end may be employed.

Although the above description has been provided in terms of the case where the first condition relates to a distance da between a diverging point Bi and the distal end of the insertion portion 11 as illustrated in Fig. 2A, a spur Spi in which the bronchus 2 diverges may be employed instead of a diverging point Bi.

(B) Case where a distance between (a position of) the distal end of the insertion portion 11 and a spur and variation of a bronchus diameter are designated as the first condition and the second condition

In this case, as illustrated in Fig. 7 instead of Fig. 2A, whether or not a distance db between a spur Spi and the distal end Pj is within a set distance dth that is a radius dth with the spur Spi as a center is performed. Note that the set distance dth for the case of a spur Spi may be set to a value that is different from the set distance dth for a diverging point Bi.

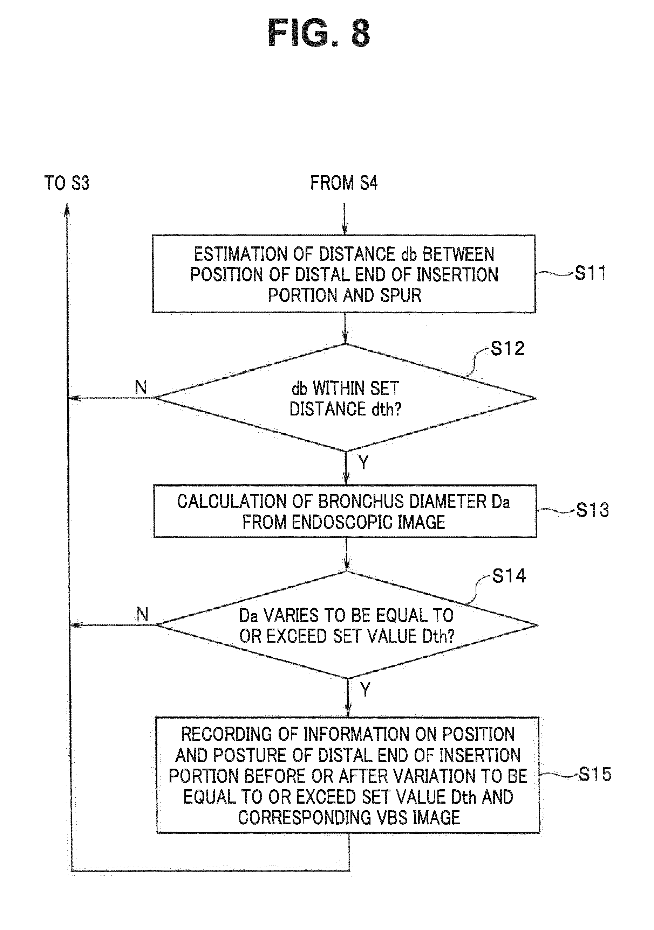

In this case, the contents of processing for the spur Spi are those provided by substituting the diverging point Bi in Figs. 4A and 4B with the spur Spi. Thus, the flowchart in Fig. 4B is changed to that illustrated in Fig. 8.

The content of step S 11 in Fig. 8 is provided by changing the distance da between the distal end position and a diverging point in Fig. 4B to a distance db between the distal end position and a spur. Also, the content of step S 12 in Fig. 8 is provided by changing da (which is the distance between the distal end position of the insertion portion 11 and the diverging point) in Fig. 4B to db (which is the distance between the distal end position of the insertion portion 11 and the spur).

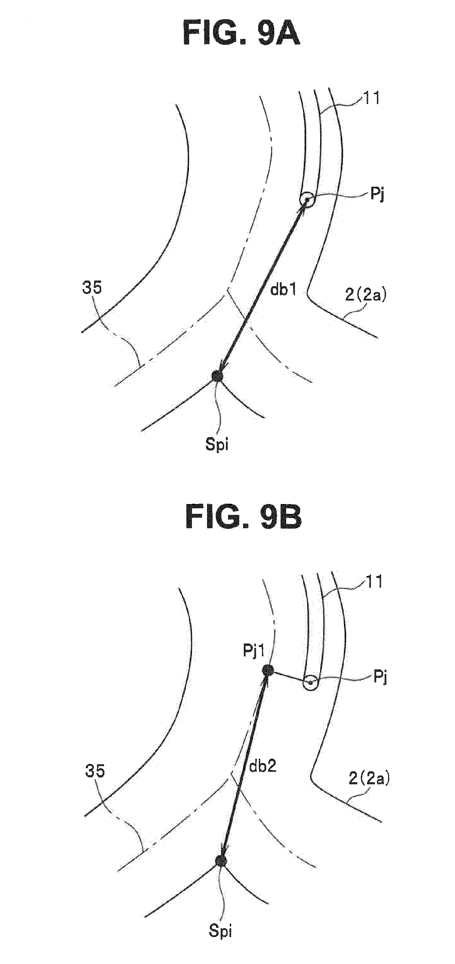

Then, operation and effects in this case are substantially the same as those of the case of the diverging point Bi. Note that as illustrated in Fig. 9A, for the distance db between the distal end position Pj of the insertion portion 11 and the spur Spi, a shortest distance db1 between the position Pj of the distal end of the insertion portion 11 and the spur Spi may be employed as the distance db. Also, as illustrated in Fig. 9B, a shortest distance db2 between a position Pj 1 on the center line 35 that is a shortest distance from the distal end position Pj of the insertion portion 11 and the spur Spi may be employed as the distance db.

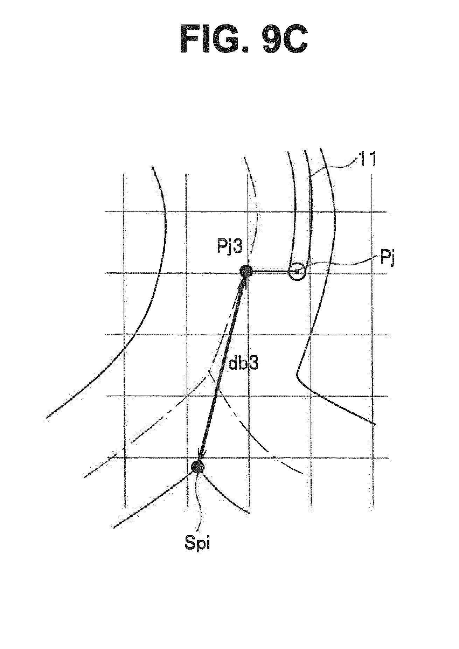

Also, as illustrated in Fig. 9C, a distance da3 between a position Pj2, which is obtained by the distal end position Pj of the insertion portion 11 being moved onto the center line 35 on the plane parallel to the coordinate plane of the CT coordinate system corresponding to the three-dimensional data of the bronchus 2, and the diverging point Bi may be employed as the distance db.

(C) Case where a distance dc between (a position of) the distal end of the insertion portion 11 and a center line 35 and variation of a bronchus diameter Da are designated as the first condition and the second condition

In this case, processing that is provided by substituting the diverging point Bi in Figs. 4A and 4B with a center line 35 is performed. In this case, when the position estimating section 25c estimates a position Pj of the distal end of the insertion portion 11, the distance comparing section 25e continuously performs operation of calculating a distance dc from the position Pj of the distal end to a center line 35 and monitoring variation of the value of the distance dc.

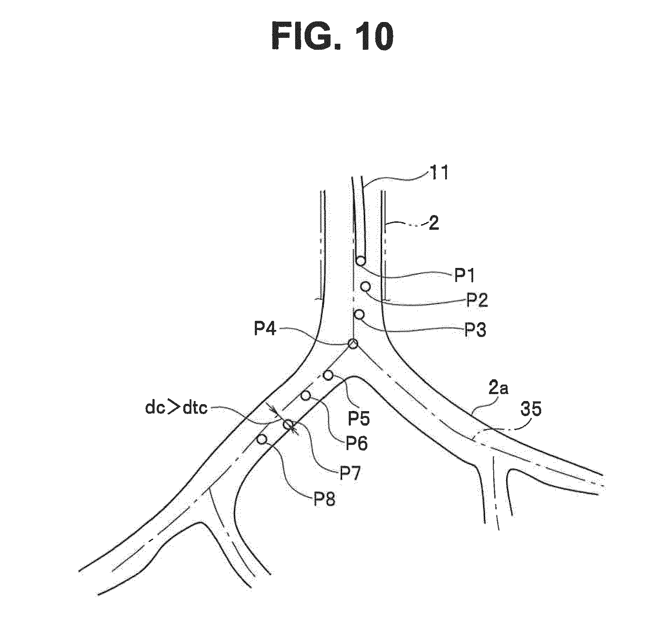

Fig. 10 illustrates estimation of positions Pj of the distal end inserted inside the bronchus 2 and determination of whether or not a first condition that the distance dc from the center line 35 to the position Pj of the distal end is within a set value dct is met. In this case, also, variation of the bronchus diameter Da is monitored. When the position Pj is moved to a current position Pj that is P8 after passing through the position P1, P2, ..., P7 over time, the distance dc is dc > dct only at the position P7.

In this case, each of the positions P1 to P6 meets the first condition and as described for case (A), the bronchus diameter Da varies by an amount that is equal to or exceeds the set value Dth when the position moves from the position P3 to the position P4 in Fig. 10, and therefore, information on the position P4 (or P3) in the vicinity of the diverging point Bi is recorded. In this case, also, operation and effects that are similar to those described for case (A) are provided.

Note that the above description has been provided in terms of the case where when the first condition is met, information is recorded if the bronchus diameter Da in the endoscopic image varies by an amount that is equal or exceeds the set value Dth, and furthermore, if variation occurs in relation to the first condition, that is, if the distance dc varies from a value that is equal to or below the set value dct to a value that is equal to or exceeds dct, information for a position Pj before the variation may be recorded.

When recording is performed as described above, an amount of information that is close to a requisite minimum can be recorded and information for a position immediately before realignment is performed can be recorded. More specifically, in the case of Fig. 10, in addition to information for the position P4 (or P3), information for the position P6, which is a position deviating from the center line 35 like the position P7, can be recorded. In this case, information for the position P6 can also be displayed as candidate information to perform realignment. Then, in this case, realignment can be performed without returning the position to the position P4.

Note that instead of monitoring the distance dc between the center line 35 and the position Pj of the distal end, a distance dd between the position Pj and a bronchus wall may be monitored. In this case, whether or not the distance dd is smaller (shorter) than a preset distance ddt may be determined.

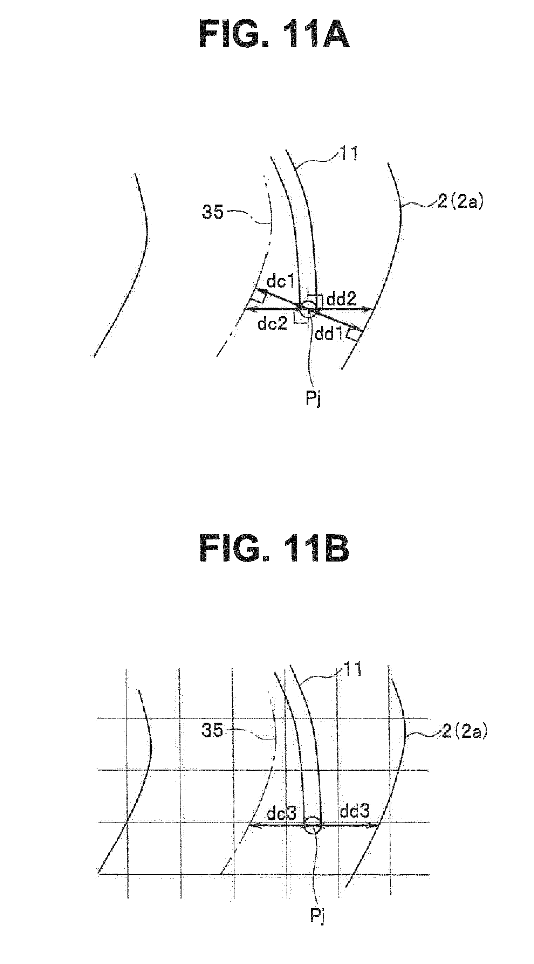

Figs. 11A and 11B illustrate calculation (measurement) of the distance dc between the position Pj of the distal end and the center line 35 or the distance dd between the position Pj of the distal end and a bronchus wall.

As illustrated in Fig. 11A, a distance dc1 from the position Pj of the distal end to the center line 35 along a line perpendicular to the center line 35 or a distance dc2 from the position Pj of the distal end to the center line 35 along a line perpendicular to the axis of the distal end may be employed as the distance dc.

Also, as illustrated in Fig. 11A, a distance dd1 from the position Pj of the distal end to the bronchus wall along a line perpendicular to the bronchus wall or a distance dd2 from the position Pj of the distal end to the bronchus wall along a line perpendicular to the axis of the distal end may be employed as the distance dd.

Also, as illustrated in Fig. 11B, a distance dc3 between the position Pj of the distal end and the center line 35 on a plane parallel to a coordinate plane in the three-dimensional data may be employed as the distance dc, or a distance dd3 between the position Pj of the distal end and the bronchus wall on the plane parallel to the coordinate plane in the three-dimensional data may be employed as the distance dd.

(D) Case where a distance de from (a position of) the distal end of the insertion portion 11 to a center of a region set in advance by a user (surgeon) and variation of a bronchus diameter Da are designated as the first condition and the second condition

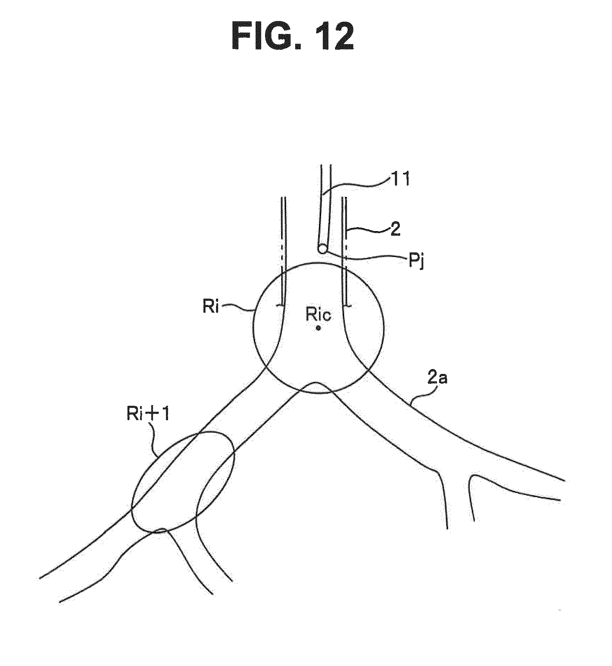

In this case, when a surgeon, which is a user, intends to insert the insertion portion 11 into a bronchus 2 via the input apparatus 31, as illustrated in Fig. 12, the surgeon sets, e.g., predetermined set regions Ri and Ri+1 in advance along a route of the insertion.

Then, the distance comparing section 25e continuously performs operation of monitoring whether or not a distance between the position Pj of the distal end of the insertion portion 11 and a center Ric of the set region Ri is within the set region Ri.

In this case, the operation is similar to that of the case where the set distance dth is set with the diverging point Bi as a center in case (A) described above. Note that not only a spherical set region Ri, but also a non-spherical set region can be set like the set region Ri+1.

The present invention is not limited to the case of the set regions having the shapes illustrated in Fig. 12, set regions having, e.g., a rectangular parallelepiped shape may be set. It is also possible that: for example, an initial region having a hemispherical shape set in advance is set with, e.g., a diverging point on a center line as a center; and after the setting, a user changes the set region to a desired size and/or shape or makes a modification such as deletion.

This case enables information recording and candidate information display (presentation) according to requests from a user.

(E) Case where a distance between a distal end position of the insertion portion 11 and a diverging point or a spur and variation of brightness of an endoscopic image are designated as the first condition and the second condition

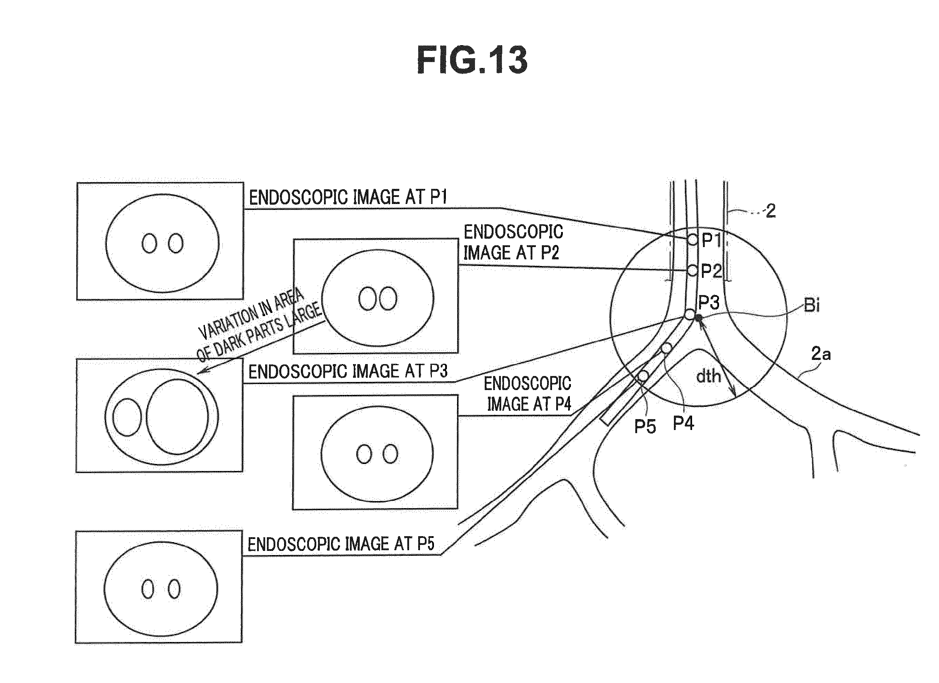

In this case, as illustrated in Fig. 13, when the insertion portion 11 is inserted into the bronchus 2, the brightness variation amount detecting section 25i in the variation amount detecting section 25g detects a variation amount of brightness of an endoscopic image.

More specifically, as indicated by positions P1, P2, ..., P5 illustrated in Fig. 13, the brightness variation amount detecting section 25i (in the image processing section 25) continuously performs operation of obtaining an endoscopic image, for example, at fixed intervals or fixed time intervals and monitoring the area of dark parts inside the obtained endoscopic image. Fig. 13 illustrates a state in which dark parts each having a value that is equal to or below a preset value together with the endoscopic images obtained for the respective positions Pj (j = 1, 2, ..., 5).

The area of dark parts refers to the total areas of image parts of an endoscopic image each having a brightness that is equal to or below a preset value. In Fig. 13, diverging parts in a lumen part on the front side of the distal end of the insertion portion 11 in the bronchus 2 are recognized as dark parts. For example, variation in area of the dark parts is small when the position moves from the position P1 to the position P2, but at the position P3 that is close to a divergence region that diverges, the area of the dark parts largely varies compared to that of the position P2. If the brightness variation amount detecting section 25i detects that the area of the dark parts has largely varied, the brightness variation amount detecting section 25i makes information including a VBS image before or after the variation be recorded in the information recording section 27.

Note that based on variation in number of diverging parts detected as dark parts (for example, variation from two to one or variation from one to two), such variation may be detected as a large variation amount of the area of the dark parts and in such case, information including a VBS image may be recorded in the information recording section 27.

The variation amount of brightness is not limited to that detected from a variation amount of the area of dark parts, and it is possible that an average value of brightness of endoscopic images is calculated and if the average value has a variation amount that is equal to or exceeds a threshold value, information including a VBS image is recorded in the information recording section 27.

The intervals for obtaining an endoscopic image may be linked with timings for obtaining the distal end position of the insertion portion 11 or may be fixed time intervals or fixed distance intervals. Furthermore, the first condition is not limited to the condition that the distance da between the distal end position and the diverging point Bi is set within the set distance dth as illustrated in Fig. 13, and a condition that the distance db between the distal end position and a spur is within a set distance may be set as the first condition.

Also, the first condition may be set using a distance other than the distances da and db.

In case (E), a variation amount for which a surgeon can easily perform comparison such as the area of dark parts is employed as a variation amount of a feature part in an image, and thus, when the image is displayed as candidate information, the surgeon can easily perform alignment visually.

(F) Case where a distance between a distal end position of the insertion portion 11 and a diverging point or a spur and variation of a divergence shape in an endoscopic image are designated as the first condition and the second condition

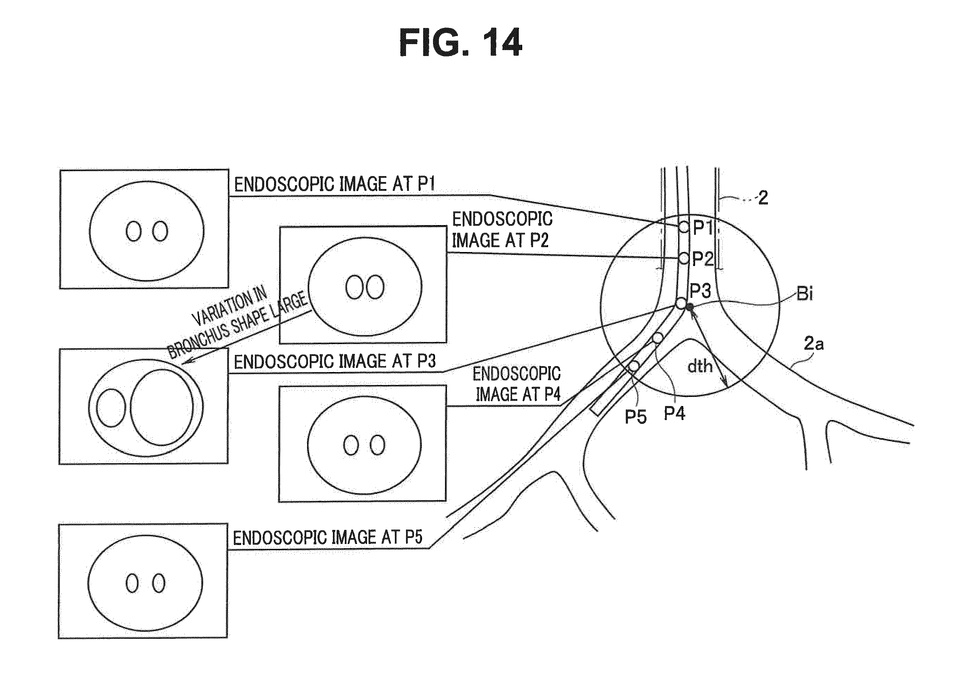

Fig. 14 illustrates extraction of a part having a divergence shape in a bronchus 2. In this case, as illustrated in Fig. 14, when the insertion portion 11 is inserted into the bronchus 2, the shape variation amount detecting section 25j in the variation amount detecting section 25g detects a variation amount of a shape of a feature part in an endoscopic image.

More specifically, for example, at fixed intervals or fixed time intervals as indicated by positions P1, P2, ..., P5 illustrated in Fig. 14, the shape variation amount detecting section 25j (in the image processing section 25) continuously performs operation of obtaining an endoscopic image and monitoring, for example, a divergence shape in the bronchus 2 in the obtained endoscopic image.

Fig. 14 illustrates extracted divergence shapes of the bronchus 2 together with the endoscopic images obtained at the respective positions Pj (j = 1, 2, ..., 5).

More specifically, variation of the bronchus divergence shape when the position moves from the position P1 to the position P2 is small, but at the position P3 that is close to a divergence region that diverges, the bronchus divergence shape largely varies compared to that at the position P2. If the shape variation amount detecting section 25j detects the large variation of the bronchus divergence shape, the shape variation amount detecting section 25j makes information including a VBS image before or after the variation be recorded in the information recording section 27.

The intervals for obtaining an endoscopic image may be linked with timings for obtaining a distal end position of the insertion portion 11, or may be fixed time intervals or fixed distance intervals. Furthermore, the first condition is not limited to the condition that the distance da between the distal end position and the diverging point Bi is within the set distance dth as illustrated in Fig. 13, a condition that the distance db between the distal end position and a spur is within a set distance may be set as the first condition.

Also, the first condition may be set using a distance other than the distances da and db.

In case (F), a variation amount for which a surgeon can easily perform comparison such as variation of a bronchus divergence shape as a variation amount of a feature part in an image, and thus, when the image is displayed as candidate information, the surgeon can easily perform alignment visually.

(G) Case where a distance between the distal end position of the insertion portion 11 and a diverging point or a spur and variation of a length of the spur in an endoscopic image are designated as the first condition and the second condition

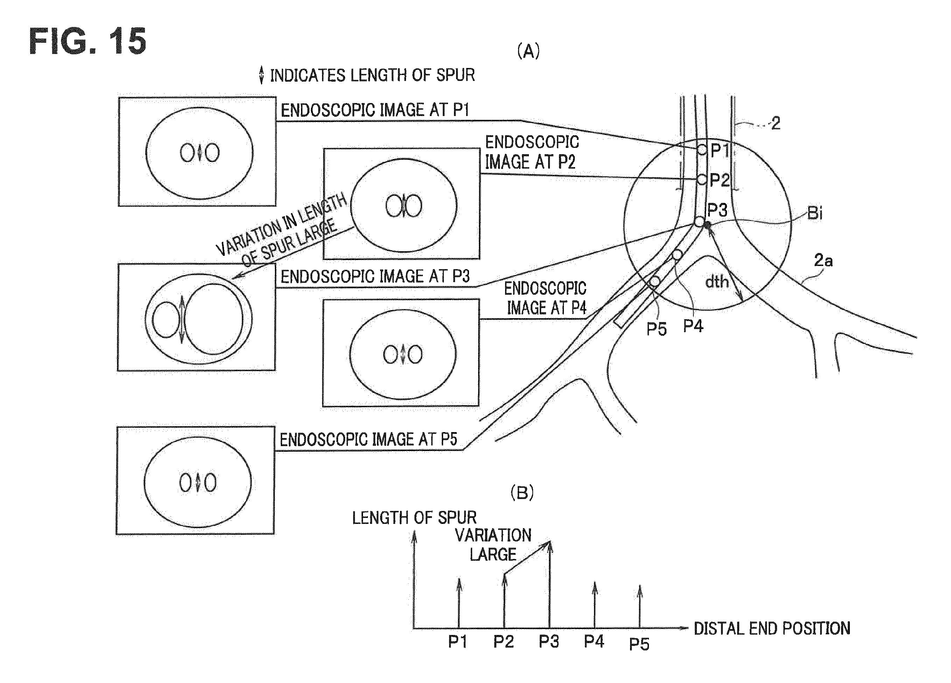

In this case, as illustrated in Fig. 15(A), when the insertion portion 11 is inserted into a bronchus 2, the spur variation amount detecting section 25k in the variation amount detecting section 25g detects a variation amount of a length of a spur in an endoscopic image. Fig. 15(A) illustrates extracted lengths of a spur together with endoscopic images obtained at respective positions Pj (j = 1, 2, ..., 5). Note that a length of a spur refers to a length of a boundary in a diverging part at which the lumen of the bronchus 2 bifurcates.

For example, at fixed intervals or fixed time intervals indicated by position P1, P2, ..., P5 illustrated in Fig. 15(A), the spur variation amount detecting section 25k (in the image processing section 25) continuously performs operation of obtaining an endoscopic image and monitoring, for example, a length of a spur in the bronchus 2 in the obtained endoscopic image. Fig. 15(B) illustrates a relationship between the position Pj of the distal end of the insertion portion 11 and the length of the spur. As can be seen from Figs. 15(A) and 15(B), for example, variation of the length of the spur when the position moves from the position P1 to the position P2 is small, but at the position P3 that is close to a divergence region that diverges, the length of the spur largely varies compared to that at the position P2. If the spur variation amount detecting section 25k detects the large variation of the length of the spur, the spur variation amount detecting section 25k makes information including a VBS image before or after the variation be recorded in the information recording section 27.

The intervals for obtaining an endoscopic image may be linked with timings for obtaining a distal end position of the insertion portion 11, or may also be fixed time intervals or fixed distance intervals. Furthermore, the first condition is not limited to the condition that the distance da between the distal end position and the diverging point Bi is within the set distance dth as illustrated in Fig. 15, and a condition that the distance db between the distal end position and a spur is within a set distance may be set as the first condition.

Also, the first condition may be set using a distance other than the distances da and db.

In case (G), a variation amount for which a surgeon can easily perform comparison such as variation of a bronchus divergence shape is employed as a variation amount of a feature part in an image, and thus, when the image is displayed as candidate information, the surgeon can easily perform alignment visually.

(H) Case where a distance between a distal end position of the insertion portion 11 and a diverging point or a spur and variation of an angle of the spur in an endoscopic image are designated as the first condition and the second condition.

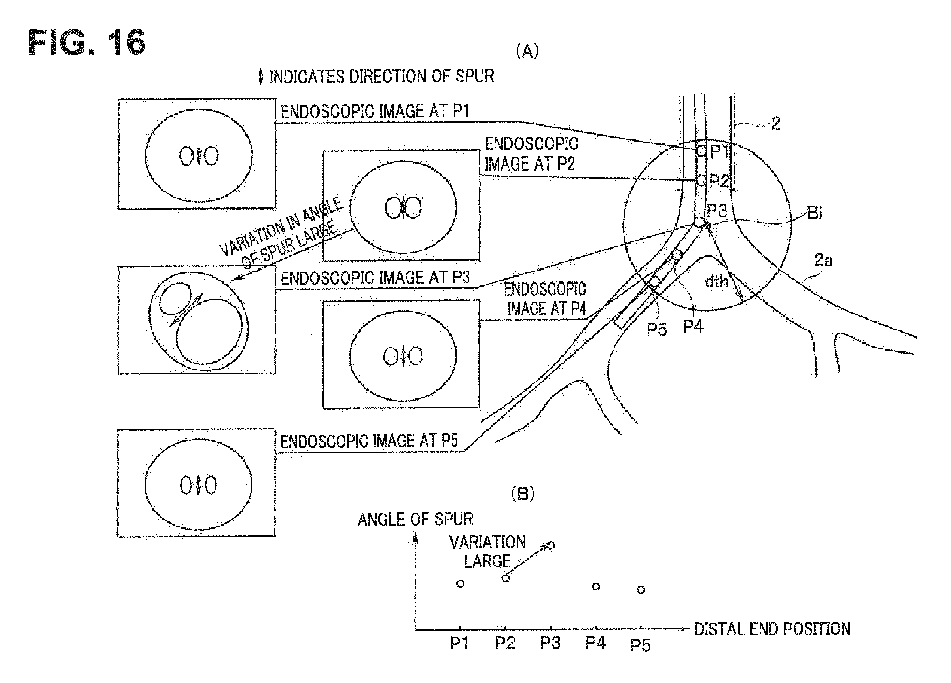

In this case, as illustrated in Fig. 16(A), when the insertion portion 11 is inserted into a bronchus 2, the spur variation amount detecting section 25k in the variation amount detecting section 25g detects a variation amount of an angle (direction) of a spur in an endoscopic image. Fig. 16(A) illustrates extracted angles of the spur together with endoscopic images obtained at respective positions Pj (j = 1, 2, ..., 5). Note that an angle of a spur refers to a direction in a longitudinal direction of a boundary part in a diverging part at which the lumen of the bronchus 2 bifurcates or an angle formed between the direction and a reference direction.

For example, at fixed intervals or fixed time intervals as indicated by positions P1, P2, ..., P5 illustrated in Fig. 16(A), the spur variation amount detecting section 25k (in the image processing section 25) continuously performs operation of obtaining an endoscopic image and monitoring, for example, an angle of a spur in the bronchus 2 in the obtained endoscopic image.

Fig. 16(B) illustrates a relationship between the position Pj of the distal end pf the insertion portion 11 and the angle of the spur. As can be seen from Figs. 16(A) and 16(B), for example, variation of the angle of the spur when the position moves from the position P1 to the position P2 is small, but at the position P3, a surgeon twists the insertion portion to be brought close to a divergence region that diverges, the angle of the spur largely varies compared to that at the position P2. If the spur variation amount detecting section 25k detects the large variation of the angle of the spur, the spur variation amount detecting section 25k makes information including a VBS image before or after the variation be recorded in the information recording section 27.

The intervals for obtaining an endoscopic image may be linked with timings for obtaining a distal end position of the insertion portion 11, or may also be fixed time intervals or fixed distance intervals. Furthermore, the first condition is not limited to the condition that the distance da between the distal end position and the diverging point Bi is within the set distance dth as illustrated in Fig. 16(A), and a condition that the distance db between the distal end position and a spur is within a set distance may be set as the first condition.

Also, the first condition may be set using a distance other than the distances da and db.

In case (H), a variation amount for which a surgeon can easily perform comparison visually such as variation of a bronchus divergence shape is employed as a variation amount of a feature part in an image, and thus, when the image is displayed as candidate information, the surgeon can easily perform alignment visually.

(I) Case where a distance between a distal end position of the insertion portion 11 and a diverging point or a spur and variation of poor visibility in an endoscopic image are designated as the first condition and the second condition

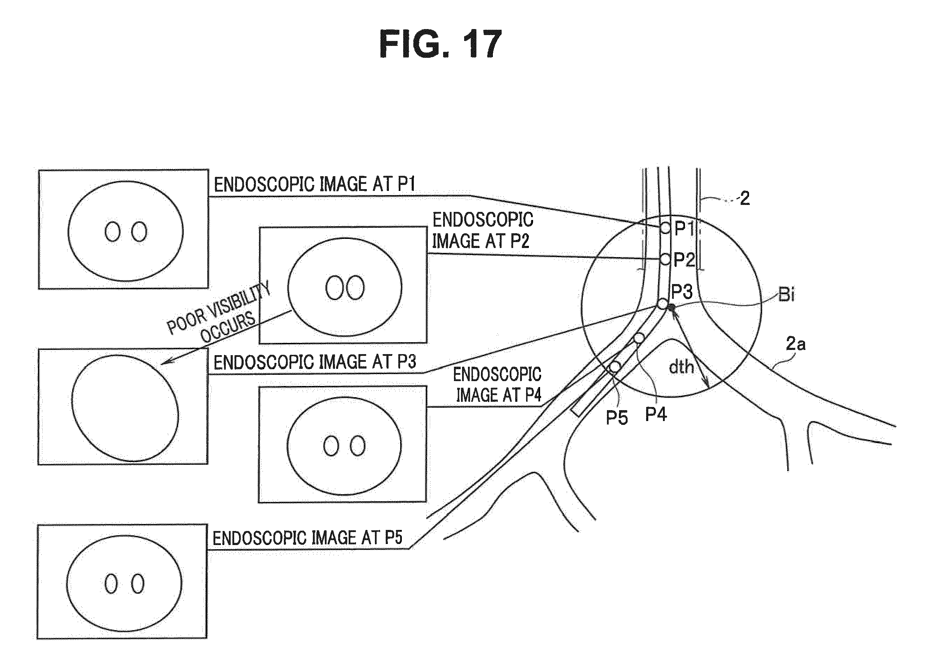

In this case, as illustrated in Fig. 17, when the insertion portion 11 is inserted into a bronchus 2, the poor visibility detecting section 251 in the variation amount detecting section 25g detects occurrence of poor visibility in an endoscopic image. The (occurrence of) poor visibility is determined by determining whether or not divergences or dark parts on the distal end side of the lumen appear to a degree that such divergences or dark parts can be recognized in an endoscopic image picked up inside the bronchus, and on the assumption that the entire view field may be covered by dirt, the poor visibility detecting section 251 determines that poor visibility occurs if a brightness of the endoscopic image becomes lower than a predetermined brightness and a dark region extends to the substantially entire endoscopic image.

Thus, for example, the brightness variation amount detecting section 25i has the function of the poor visibility detecting section 251.

Fig. 17 illustrates an overview of endoscopic images obtained at respective positions Pj (j = 1, 2, ..., 5). For example, at fixed intervals or fixed time intervals indicated by the positions P1, P2, ..., P5, the poor visibility detecting section 251 (in the image processing section 25) continuously performs operation of obtaining an endoscopic image and monitoring poor visibility in the obtained endoscopic image. In the example illustrated in Fig. 17, the poor visibility detecting section 251 detects occurrence of poor visibility when the position moves from the position P2 to the position P3, and makes information including a VBS image for the position P2 immediately before the variation be recorded in the information recording section 27.

The intervals for obtaining an endoscopic image may be linked with timings for obtaining a distal end position of the insertion portion 11, or may also be fixed time intervals or fixed distance intervals. Furthermore, the first condition is not limited to the condition that the distance da between the distal end position and the diverging point Bi is within the set distance dth as illustrated in Fig. 16(A), and a condition that the distance db between the distal end position and a spur is within a set distance may be set as the first condition. Also, the first condition may be set using a distance other than the distances da and db.

In case (I), a variation amount for which a surgeon can easily perform comparison visually such as poor visibility is employed as a variation amount of a feature part in an image, and thus, the surgeon can easily grasp a state for which information was recorded.

Note that although the above-described shape variation amount detecting section 25j detects a variation amount of a divergence shape inside a bronchus, upon variation from a divergence shape to a structure or a shape other than the divergence shape, in other words, upon detection of variation to a structure or a shape other than a divergence shape, information may be recorded.

(J) Case where a distance between a distal end position of the insertion portion 11 and a diverging point or a spur and variation of a bronchus divergence shape in an endoscopic image are designated as the first condition and the second condition

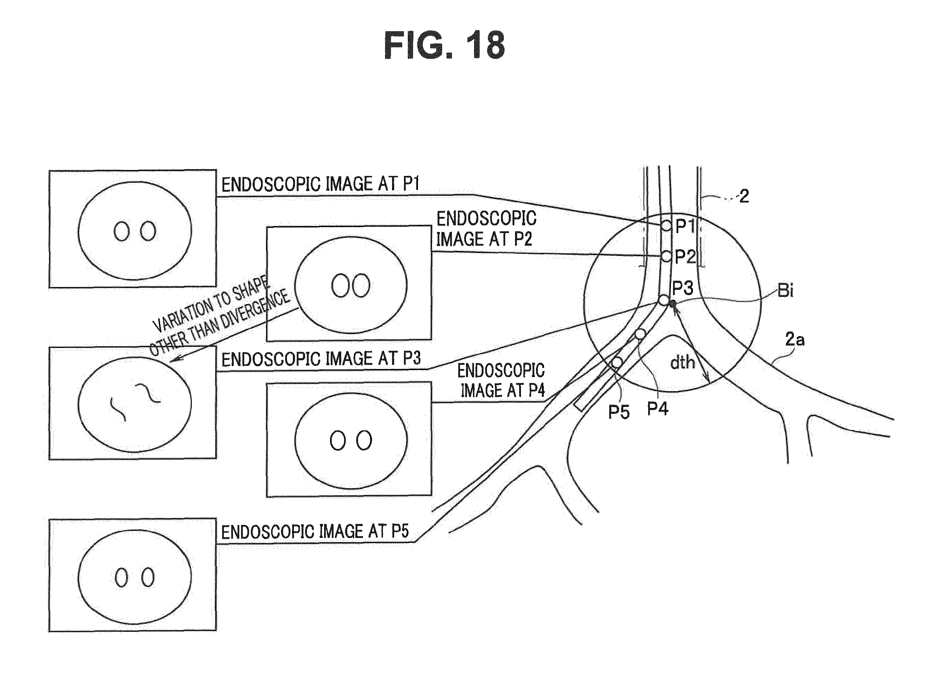

In this case, as illustrated in Fig. 18, when the insertion portion 11 is inserted to a bronchus 2, the shape variation amount detecting section 25j in the variation amount detecting section 25g continuously performs operation of monitoring whether or not a divergence of the bronchus 2 exists in an endoscopic image obtained by image pickup of the inside of the bronchus 2. Then, if it is determined that no divergence exists in the endoscopic image as a result of the bending portion 19 of the insertion portion 11 being bent by, or the insertion portion 11 being twisted by, a surgeon, information including a VBS image for a position immediately before the shape variation is recorded in the information recording section 27.

As illustrated in Fig. 18, for example, at fixed intervals or fixed time intervals as indicated by position P1, P2, ..., P5, the shape variation amount detecting section 25j (in the image processing section 25) continuously performs operation of obtaining an endoscopic image, extracting, for example, a divergence shape part in the obtained endoscopic image and monitoring whether or not the divergence exists. Then, when the position moves from the position P2 to the position P3 in Fig. 18, the shape variation amount detecting section 25j determines that the state has changed to a state in which the divergence does not exist, and makes information including a VBS image for the position P2 immediately before the change be recorded in the information recording section 27.

The intervals for obtaining an endoscopic image may be linked with timings for obtaining a distal end position of the insertion portion 11, or may also be fixed time intervals or fixed distance intervals. Furthermore, the first condition is not limited to the condition that the distance da between the distal end position and the diverging point Bi is within the set distance dth as illustrated in Fig. 16(A), and a condition that a distance db between the distal end position and a spur is within a set distance may be set as the first condition. Also, the first condition may be set using a distance other than the distances da and db.

In case (J), a variation amount for which a surgeon can easily perform comparison visually such as existence or non-existence of a divergence shape is employed as a variation amount of a feature part in an image, and thus, the surgeon can easily grasp a state for which information was recorded.

(K) Case where a distance between a distal end position of the insertion portion 11 and a diverging point or a spur and variation of displacement of a feature part in an endoscopic image are designated as the first condition and the second condition

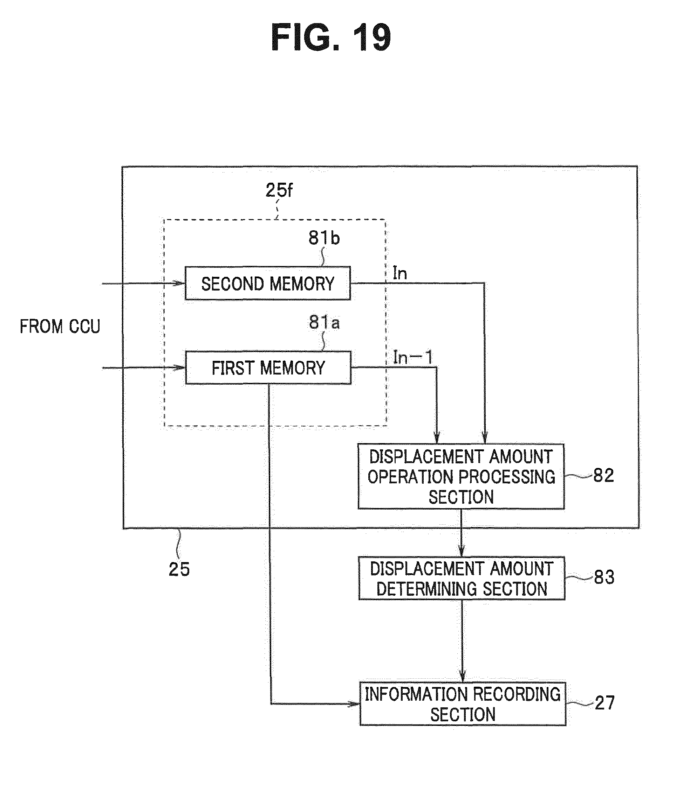

In this case, as illustrated in Fig. 19, the image processing section 25 stores image signals sequentially inputted at predetermined time intervals (for example, 1/30s or 1/60s) from the CCU 8A, alternately in a first memory 81a and a second memory 81b in the image memory 25f. For example, a latest n-th image In is stored in the second memory 81b, and an n-1-th image In-1 one frame or one field before the n-th image is stored in the second memory 81b.

The n-1-th image In-1 and the n-th image In picked up in frames or fields adjacent to each other are inputted to a displacement amount operation processing section 82, and the displacement amount operation processing section 82 performs an operation to calculate a motion vector quantity indicating an amount of displacement of a point in one image corresponding to a point set in the other image (for example the image In) from the point set in the other image.

The motion vector quantity calculated by the displacement amount operation processing section 82 is inputted to a displacement amount determining section 83, and the displacement amount determining section 83 recognizes the calculated motion vector quantity as a displacement amount and determines whether or not a magnitude (absolute value) of the motion vector quantity exceeds a preset value, and according to a result of the determination, makes information including a VBS image be recorded as candidate information. Note that a configuration in which the condition determining section 26b illustrated in Fig. 1 has the function of the displacement amount determining section 83 may be employed.

The displacement amount operation processing section 82 sets a range of W x H pixels with a center point of the image In as a center, as a template, and finds a corresponding point in the image In-1 corresponding to the center point. The finding of the corresponding point is performed by calculating, for example, a SAD (sum of absolute differences) in luminance. Where t(x, y) is a pixel value in the template and g(x, y) is a pixel value in the image that is the target of the finding, F(u, v), which is a SAD in coordinates (u, v), can generally be calculated according to Expression (4).

$$F(u, v) = \Sigma_i \Sigma_j |g(i+u, j+v) - t(i,j)| \qquad (4)$$

$\Sigma_i$ and $\Sigma_j$ indicate that an operation to add up |g-t| for a width W and a height H of the template in which i is within $N_W$ and within $N_H$, respectively, and W is the width of the template and H is the height of the template, and $-W/2 \leq N_W \leq W/2$, and $-H/2 \leq N_H \leq H/2$. Also, (Ox, Oy) are central coordinates in the image In-1 corresponding to the image In, and F(u, v) is calculated within the ranges of $Ox-W/2 \leq u \leq Ox+W/2$ and $Oy-H/2 \leq v \leq Oy+H/2$. Coordinates (Ex, Ey) when F(u, v) is minimum provide the corresponding point.

From the coordinates (Ex, Ey) of the corresponding point for the central coordinates (Ox, Oy) in the image In, a motion vector m is calculated according to Expression (5).

$$m = (Ex-Ox, Ey-Oy) \ldots (5)$$

The motion vector m is calculated according to the method described above.

Upon the processing for calculating the motion vector m as a displacement amount being ended by the calculation of the motion vector m, the displacement amount determining section 83 compares the magnitude of the motion vector m with the preset value, and if the displacement amount determining section 83 determines that the magnitude of the motion vector m is larger than the preset value, the displacement amount determining section 83 determines that variation that is a displacement exceeding the preset value occurs for the information recording section 27, and outputs a recording instruction signal (or storage instruction signal) for recording a VBS image to the information recording section 27. Upon receipt of the recording instruction signal, the information recording section 27 records the image before occurrence of the displacement exceeding the preset value from the first memory 81a of the image memory 25f, as a candidate image. The operation of recording a candidate image is performed each time a recording instruction signal is inputted, and thereby candidate images are accumulated in the information recording section 27.

Repetition of the above operation enables endoscopic images immediately before endoscopic images whose displacement is larger than the preset value to be accumulated as candidate images. For a method of detecting a displacement of an endoscopic image, an operation according to SHIFT (scale-invariant feature transform) may be employed, and if an operation to calculate a feature point corresponding to each image fails or if a frequency analysis of an image indicates that high-frequency components are reduced by an amount that is equal to or exceeds a preset value, recording may be performed in such a manner as described above. In such case, effects similar to the above can also be provided.

Note that the above embodiment has been described in terms of typical combinations of a first condition and a second condition, information may be recorded according to a combination other than the above-described combinations.

In other words, the present invention includes configurations and methods using a combination of a first condition and a second condition that is different from any of those used in the present embodiment. Furthermore, the first condition and the second condition are set not only by a user, but also may be set by recording the first condition and the second condition in advance in, for example, the condition setting section 26d or the information recording control section 26c in the apparatus without a user setting the first condition and the second condition.

Also, the condition information recording section 27a, which is condition information recording means, has been described as recoding information on a plurality of candidate conditions that can be set as the first condition and the second condition and candidate information, respectively, the condition information recording section 27a, may be described as recording a plurality of condition information pieces (or information pieces) that can be set as the first condition and the second condition, respectively, without using information on candidate conditions and candidate information.

In the above description, if an instruction signal for realignment is inputted to the control section 26 via, e.g., the input apparatus 31, the information recorded in the information recording section 27 is displayed (presented) as candidate information on the monitor 32, which is display means.

The present invention is not limited to this case, and for example, information recorded at a predetermined timing in the information recording section 27 may be displayed (represented) as candidate information on the monitor 32, which is display means.

For example, arrangement may be made so that a user provides an input for setting a time interval or a condition for displaying candidate information to the control section 26 via, e.g., the input apparatus 31, and the control section 26 performs control to if the set time interval or condition is met, read information from the information recording section 27 to display the candidate information including VBS images on the monitor 32 via the image processing section 25.

Also, in a configuration including image comparing means for comparing information on an image picked up by image pickup means and a virtual endoscopic image, and display means for displaying, at predetermined timing, a virtual endoscopic image recorded in information obtaining means, the information obtaining means may be configured to obtain at least position information on the image pickup means based on a result of the comparison by the image comparing means.

Also, the present invention is not limited to the above-described configuration illustrated in, for example, Fig. 1, and only the basic configuration stated in claim 1 may be employed, and a configuration obtained by selectively adding one or more components to this basic configuration may be employed.

The present application is filed claiming the priority of Japanese Patent Application No. 2013-044601 filed in Japan on March 6, 2013, and the above described disclosure is incorporated by reference in the present description and claims.

## Claims

1. An endoscope system comprising:

   an image recording section that records three dimensional image information of a subject obtained in advance;
   a luminal organ extracting section that extracts a predetermined luminal organ from the three dimensional image information;
   a virtual endoscopic image generating section that generates a virtual endoscopic image, the virtual endoscopic image being endoscopically drawn from a predetermined viewpoint position with respect to information of the predetermined luminal organ extracted by the luminal organ extracting section;
   an image pickup section provided inside an endoscope, the image pickup section picking up an image of an inside of the predetermined luminal organ;
   a position information obtaining section that obtains information on a position of a distal end of an insertion portion of the endoscope as position information;
   a distance comparing section that compares a distance from a feature region in the predetermined luminal organ extracted by the luminal organ extracting section to the position of the distal end of the insertion portion of the endoscope obtained by the position information obtaining section with a set distance;
   a variation amount detecting section that detects a variation amount of a feature part of the predetermined luminal organ in the endoscopic image picked up by the image pickup section; and
   an information recording section that records position and image information including the position of the distal end of the insertion portion of the endoscope and the virtual endoscopic image corresponding to the position of the distal end based on a result of the comparison by the distance comparing section and a result of the detection by the variation amount detecting section.

2. The endoscope system according to claim 1, wherein the distance comparing section compares a first distance or a second distance with the set distance, the first distance being from the position of the distal end of the insertion portion of the endoscope to a divergence region of the predetermined luminal organ in which the lumen diverges, the second distance being between the position of the distal end of the insertion portion of the endoscope and a center line passing through a center of the predetermined luminal organ.

3. The endoscope system according to claim 1, wherein the variation amount detecting section detects a variation amount of a shape of the feature part in a divergence region of the predetermined luminal organ in which the lumen diverges in the endoscopic image.

4. The endoscope system according to claim 1, wherein the variation amount detecting section detects a variation amount of brightness of the feature part in a divergence region of the predetermined luminal organ in which the lumen diverges in the endoscopic image.

5. The endoscope system according to claim 1, further comprising a condition determining section that determines whether or not the result of the comparison by the distance comparing section and the result of the detection by the variation amount detecting section meet respective predetermined conditions including a first condition and a second condition, wherein
if the condition determining section determines that the first condition and the second condition are met, the information recording section records the position and the image information.

6. The endoscope system according to claim 1, further comprising an image comparing section that compares the endoscopic image picked up by the image pickup section with the virtual endoscopic image generated by the virtual endoscopic image generating section,
wherein the position information obtaining section obtains the position information of the distal end of the insertion portion of the endoscope based on a result of comparison by the image comparing section.

7. The endoscope system according to claim 6, further comprising:

   a lumen shape image generating section that generates a lumen shape image of the predetermined luminal organ; and
   a display control section that if the position information obtaining section fails to obtain information on the position of the distal end of the insertion portion of the endoscope that is based on the result of the comparison by the image comparing section or an instruction signal for presenting the position and the image information recorded in the information recording section is generated, performs control so as to display the position of the distal end of the insertion portion of the endoscope at the position and the image information recorded in the information recording section at a corresponding position in the lumen shape image, and display the virtual endoscopic image corresponding to the position of the distal end,
   wherein the position information obtaining section obtains the position information of the distal end of the insertion portion of the endoscope by comparing the virtual endoscopic image read from the information recording section with a current endoscopic image picked up by the image pickup section.

8. The endoscope system according to claim 5, further comprising:

   a condition information recording section that records a plurality of condition information pieces that can be selectively set as the first condition and the second condition, respectively; and
   a designation section that selectively designates condition information pieces to be used as the first condition and the second condition, respectively, from the condition information recording section.

9. The endoscope system according to claim 1, further comprising:

   an input section that generates an instruction signal for presenting the position and the image information recorded in the information recording section; and
   a display apparatus that displays the position and the image information recorded in the information recording section based on the generation of the instruction signal.

10. The endoscope system according to claim 1, wherein
the variation amount detecting section estimates an inner diameter of a lumen as the feature part of the predetermined luminal organ in the endoscopic image every fixed period of time and thereby detects a variation amount of the inner diameter in the fixed period of time, and
the information recording section records the position and the image information where the variation amount detecting section detects a variation amount of the inner diameter that is equal to or exceeds a set value.

11. The endoscope system according to claim 5, wherein
the variation amount detecting section estimates an inner diameter of a lumen as the feature part of the predetermined luminal organ in the endoscopic image every fixed period of time and thereby detects a variation amount of the inner diameter in the fixed period of time, and
if the variation amount detecting section detects a variation amount of the inner diameter that is equal to or exceeds a set value, the condition determining section determines that the first condition is met.

**12.** The endoscope system according to claim 5, wherein
the variation amount detecting section includes at least one of a brightness variation amount detecting section that detects a variation amount of brightness of a divergence region in which a lumen diverges, as the feature part of the predetermined luminal organ in the endoscopic image, and a shape variation amount detecting section that detects a variation amount of a shape of the divergence region, and
if the variation amount detecting section detects a variation amount of the brightness or the shape that is equal to or exceeds a set value, the condition determining section determines that the second condition is met.

**13.** The endoscope system according to claim 5, wherein
the distance comparing section compares a first distance from the position of the distal end of the insertion portion of the endoscope to a divergence region of the predetermined luminal organ in which the lumen diverges, a second distance between the position of the distal end of the insertion portion of the endoscope and a center line extending through a center of the predetermined luminal organ or a third distance between the position of the distal end of the insertion portion of the endoscope and a center line diverging point at which the center line extending through the center of the predetermined luminal organ diverges, the first distance, the second distance or the third distance being obtained every fixed period of time by the position information obtaining section, with the set distance set for the first distance, the second distance or the third distance, and
if the distance comparing section determines that the first distance, the second distance or the third distance is within the set distance, the condition determining section determines that the first condition is met.

**14.** The endoscope system according to claim 13, wherein if the condition determining section determines that the first condition is met, the information recording section further records information on an axis direction of the distal end of the insertion portion as the position and image information including the position of the distal end of the insertion portion and the virtual endoscopic image.

**15.** The endoscope system according to claim 12, wherein if the condition determining section determines that the second condition is met, the information recording section further records information on an axis direction of the distal end of the insertion portion as the position and image information including the position of the distal end of the insertion portion and the virtual endoscopic image.

# FIG. 1

INSERTION SUPPORT APPARATUS
IMAGE PROCESSING SECTION
ALIGNMENT PROCESSING SECTION
IMAGE COMPARING SECTION
POSITION ESTIMATING SECTION
IMAGE GENERATING SECTION
DISTANCE COMPARING SECTION
VARIATION AMOUNT DETECTING SECTION
IMAGE MEMORY
CONTROL SECTION
DISPLAY CONTROL SECTION
CONDITION DETERMINING SECTION
INFORMATION RECORDING CONTROL SECTION
CONDITION SETTING SECTION
INFORMATION RECORDING SECTION
CONDITION INFORMATION RECORDING SECTION
VBS IMAGE GENERATING SECTION
BRONCHUS EXTRACTING SECTION
BRONCHUS SHAPE IMAGE GENERATING SECTION
ROUTE SETTING SECTION
MPR IMAGE GENERATING SECTION
CT IMAGE DATA RECORDING SECTION
CT DATA LOADING SECTION
INPUT APPARATUS
MONITOR
CCU
LIGHT SOURCE APPARATUS
1
2a
32
31
31a
26
26a
26b
26c
26d
29
29a
28
25
25a
25b
25c
25d
25e
25f
25g
25h
25i
25j
25k
25ℓ
27
27a
24
23
23a
23b
22
22a
21
5
4A
9A
8A
6
3A
20
12
11
14
19
13
7
15
16

# FIG. 2A

11
Pj
2
da
Bi
2a
35
dth
Bi+1
Pk
K
Bi+2
36

# FIG. 2B

11
2
P1
P2
P3
P4
2a
P7
P8
dth
P6
P5

# FIG. 2C

BRONCHUS
DIAMETER Da
VARIATION LARGE
Dth
P1
P2
P3
P4
P5
P6
BRONCHUS DIAMETER
CALCULATION POSITION

# FIG. 2D

BVS IMAGE
ENDOSCOPIC IMAGE
CURRENT ENDOSCOPIC IMAGE
BVS IMAGE
ENDOSCOPIC IMAGE
2
Bi
2a
Bi+1
Pk

4B
6
LIGHT SOURCE APPARATUS
18b
IMAGE PICKUP CONTROL SECTION
18a
IMAGE PICKUP CONTROL SECTION
MEASUREMENT OPERATING SECTION
18d
TO IMAGE PROCESSING SECTION 25
18c
STEREO IMAGE SIGNAL GENERATING SECTION
9B
STEREO DISPLAY MONITOR
8B
11
13
14
3B
7a
15a
7b
15b
16a
16b
16'

FIG. 3A

# FIG. 3B

z
60
(X, Y, Z)
x
64
D
0
63
OR
62(XR, YR)
F
OL
61(XL, YL)
PL
y

# FIG. 3C

71
72
74
73

# FIG. 3D

75
Da
60a
60b

# FIG. 3E

60'
16a'
15a'
7a'
D'
150'
7b'
16b'
13
63'
64'
61'
62'
19
2
11(3A)

# FIG. 4A

START
INITIAL SETTINGS
(SETTING OF FIRST CONDITION
AND SECOND CONDITION)
S1
ALIGNMENT BY MEANS OF IMAGE MATCHING
S2
ESTIMATION (OBTAINMENT) OF POSITION
AND POSTURE OF DISTAL END OF
INSERTION PORTION
S3
S4
INSTRUCTION
SIGNAL FOR REALIGNMENT
PROVIDED?
Y
N
S7
DISPLAY CANDIDATE
INFORMATION PIECES
S8
REALIGNMENT
S5
N
PREDETERMINED
CONDITIONS MET?
Y
RECORD INFORMATION ON CORRESPONDING
VBS IMAGE TOGETHER WITH POSITION AND
POSTURE OF DISTAL END OF
INSERTION PORTION
S6

# FIG. 4B

TO S3
FROM S4
ESTIMATION OF DISTANCE da BETWEEN POSITION OF DISTAL END OF INSERTION PORTION AND DIVERGING POINT
S11
S12
N
da WITHIN SET DISTANCE dth?
Y
CALCULATION OF BRONCHUS DIAMETER Da FROM ENDOSCOPIC IMAGE
S13
S14
N
Da VARIES TO BE EQUAL TO OR EXCEED SET VALUE Dth?
Y
RECORDING OF INFORMATION ON POSITION AND POSTURE OF DISTAL END OF INSERTION PORTION BEFORE OR AFTER VARIATION TO BE EQUAL TO OR EXCEED SET VALUE Dth AND CORRESPONDING VBS IMAGE
S15

# FIG. 5

(A)
11
Pj
35
da = SHORTEST DISTANCE
Bi
(B)
11
35
Pj1
Pj
da1
Bi

(C)
11
35
Pj2
Pj
da2
Bi
PL

# FIG. 6

11
2(2a)
35
Da2
Da1

# FIG. 7

11
Pj
2
da
Bi
2a
35
dth
Bi+1
Spi
Bi+2
Spi+2
Spi+1

# FIG. 8

TO S3
FROM S4
ESTIMATION OF DISTANCE db BETWEEN POSITION OF DISTAL END OF INSERTION PORTION AND SPUR
S11
S12
db WITHIN SET DISTANCE dth?
N
Y
CALCULATION OF BRONCHUS DIAMETER Da FROM ENDOSCOPIC IMAGE
S13
S14
Da VARIES TO BE EQUAL TO OR EXCEED SET VALUE Dth?
N
Y
RECORDING OF INFORMATION ON POSITION AND POSTURE OF DISTAL END OF INSERTION PORTION BEFORE OR AFTER VARIATION TO BE EQUAL TO OR EXCEED SET VALUE Dth AND CORRESPONDING VBS IMAGE
S15

# FIG. 9A

11
Pj
35
db1
2(2a)
Spi

# FIG. 9B

11
Pj1
Pj
35
db2
2(2a)
Spi

# FIG. 9C

11
Pj3
Pj
db3
Spi

# FIG. 10

11
2
P1
P2
P3
P4
2a
35
dc>dtc
P5
P6
P7
P8

# FIG. 11A

11
35
2(2a)
dc1
dd2
dc2
dd1
Pj

# FIG. 11B

11
35
2(2a)
dc3
dd3
Pj

# FIG. 12

11
2
Pj
Ri
Ric
2a
Ri+1

FIG.13

2
2a
Bi
P1
P2
P3
P4
P5
dth
ENDOSCOPIC IMAGE AT P1
ENDOSCOPIC IMAGE AT P2
ENDOSCOPIC IMAGE AT P3
ENDOSCOPIC IMAGE AT P4
ENDOSCOPIC IMAGE AT P5
VARIATION IN AREA OF DARK PARTS LARGE

# FIG. 14

2
2a
Bi
P1
P2
P3
P4
P5
dth
ENDOSCOPIC IMAGE AT P1
ENDOSCOPIC IMAGE AT P2
ENDOSCOPIC IMAGE AT P3
ENDOSCOPIC IMAGE AT P4
ENDOSCOPIC IMAGE AT P5
VARIATION IN BRONCHUS SHAPE LARGE

FIG. 15

(A)
↕ INDICATES LENGTH OF SPUR
ENDOSCOPIC IMAGE AT P1
ENDOSCOPIC IMAGE AT P2
ENDOSCOPIC IMAGE AT P3
ENDOSCOPIC IMAGE AT P4
ENDOSCOPIC IMAGE AT P5
VARIATION IN LENGTH OF SPUR LARGE
P1
P2
P3
P4
P5
Bi
2a
2
dth
(B)
LENGTH OF SPUR
VARIATION LARGE
DISTAL END POSITION

FIG. 16
(A)
INDICATES DIRECTION OF SPUR
ENDOSCOPIC IMAGE AT P1
ENDOSCOPIC IMAGE AT P2
VARIATION IN ANGLE OF SPUR LARGE
ENDOSCOPIC IMAGE AT P3
ENDOSCOPIC IMAGE AT P4
ENDOSCOPIC IMAGE AT P5
2
P1
P2
P3
Bi
2a
dth
P5
P4
(B)
ANGLE OF SPUR
VARIATION LARGE
DISTAL END POSITION
P1
P2
P3
P4
P5

# FIG. 17

2
2a
Bi
P1
P2
P3
P4
P5
dth
ENDOSCOPIC IMAGE AT P1
ENDOSCOPIC IMAGE AT P2
ENDOSCOPIC IMAGE AT P3
ENDOSCOPIC IMAGE AT P4
ENDOSCOPIC IMAGE AT P5
POOR VISIBILITY OCCURS

# FIG. 18

ENDOSCOPIC IMAGE AT P1
ENDOSCOPIC IMAGE AT P2
VARIATION TO SHAPE OTHER THAN DIVERGENCE
ENDOSCOPIC IMAGE AT P3
ENDOSCOPIC IMAGE AT P4
ENDOSCOPIC IMAGE AT P5
2
P1
P2
P3
Bi
2a
dth
P4
P5

# FIG. 19

25f
81b
SECOND MEMORY
In
FROM CCU
81a
FIRST MEMORY
In−1
DISPLACEMENT AMOUNT OPERATION PROCESSING SECTION
82
25
DISPLACEMENT AMOUNT DETERMINING SECTION
83
INFORMATION RECORDING SECTION
27

**INTERNATIONAL SEARCH REPORT**

International application No. PCT/JP2014/053875

A. CLASSIFICATION OF SUBJECT MATTER
*A61B1/00*(2006.01)i, *A61B1/267*(2006.01)i, *A61B1/273*(2006.01)i

According to International Patent Classification (IPC) or to both national classification and IPC

B. FIELDS SEARCHED

Minimum documentation searched (classification system followed by classification symbols)
A61B1/00, A61B1/267, A61B1/273

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2011-212244 A (Fujifilm Corp.), 27 October 2011 (27.10.2011), paragraphs [0017], [0023], [0039]; 7th carrying-out mode & US 2013/23730 A1 & EP 2554103 A1 & CN 102821671 A | 1-15 |
| A | WO 2011/94518 A2 (THE PENN STATE RESEARCH FOUNDATION), 04 August 2011 (04.08.2011), & JP 2013-517909 A & EP 2528496 A1 & CN 102883651 A | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 April, 2014 (15.04.14) | 28 April, 2014 (28.04.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/053875

C (Continuation). DOCUMENTS CONSIDERED TO BE RELEVANT

| Category* | Citation of document, with indication, where appropriate, of the relevant passages | Relevant to claim No. |
|---|---|---|
| A | JP 2012-505695 A (Koninklijke Philips Electronics N.V.), 08 March 2012 (08.03.2012), abstract; paragraph [0004] & US 2011/282151 A1 & EP 2348954 A1 & CN 102186404 A | 1-15 |
| A | WO 2011/101754 A1 (KONINKLIJKE PHILIPS ELECTRONICS N.V.), 25 August 2011 (25.08.2011), fig. 1 & JP 2013-519486 A & US 2012/302878 A1 & CN 102858229 A | 1-15 |
| A | JP 2005-338551 A (Olympus Corp.), 08 December 2005 (08.12.2005), abstract; paragraphs [0035], [0081], [0093] (Family: none) | 1-15 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007129493 A
- JP 2011212244 A
- JP 2011027911 A
- JP 2013044601 A